# EUROPEAN PATENT APPLICATION

(11) **EP 4 578 871 A1**
(43) Date of publication of application: **02.07.2025**
(21) Application number: 23857675.5
(22) Date of filing: 21.08.2023
(51) Int. Cl.: C07K 14/55, C07K 16/28, C07K 16/30, A61K 38/00, A61P 35/00, A61K 39/00

(54) **IL2 VARIANT AND PROTEIN COMPLEX COMPRISING SAME**

(30) Priority: 23.08.2022 KR 20220105643; 02.05.2023 KR 20230057361
(71) Applicant: Mustbio Co., Ltd., Seoul 08390 (KR)
(72) Inventor: BANG, Hyo Joo, Ansan-si, Gyeonggi-do 15578 (KR); JUNG, Yong Jun, Suwon-si, Gyeonggi-do 16509 (KR); JUNG, Sung Youb, Suwon-si, Gyeonggi-do 16507 (KR); PARK, Young Jin, Suwon-si, Gyeonggi-do 16509 (KR); KANG, Seok Chan, Bucheon-si, Gyeonggi-do 14547 (KR); PARK, Bom, Suwon-si, Gyeonggi-do 16505 (KR); PARK, Sang Hyun, Suwon-si, Gyeonggi-do 16531 (KR); CHO, Sun Jung, Seongnam-si, Gyeonggi-do 13611 (KR)
(74) Representative: EIP
(86) International application number: PCT/KR2023/012348
(87) International publication number: WO 2024/043643

(57) **Abstract**

Provided are an IL2 variant and a protein complex including the same, and a production method and use thereof. The protein complex may reduce side effects and maximize anticancer activity by selectively increasing effector T cell activity, and thus the protein complex may be used for preventing or treating various cancer-related immunological diseases.

## Description

### Technical Field

This application claims the benefit of Korean Patent Application No. 10-2022-0105643 filed on August 23, 2022 and Korean Patent Application No. 10-2023-0057361 filed on May 2, 2023, in the Korean Intellectual Property Office, the disclosures of which are incorporated herein in their entirety by reference.

The disclosure relates to IL2 variants and protein complexes including the same, production methods therefor, and uses thereof.

### Background Art

Immune checkpoints refer to surface proteins of immune cells used by cancer cells to evade attacks from the immune system, and cancer cells bind to PD-1, which is one of the immune checkpoints of T cells, by expressing PD-L1 on the surfaces thereof, thereby inhibiting activation of T cells and cancer cell-killing ability thereof. Immune checkpoint inhibitors may solve some side effects and resistance problems of existing anticancer drugs that directly attack cancer cells, by allowing immune cells to selectively attack cancer cells by activating the immune system in the human body. In addition, many immune checkpoint inhibitors have been approved for sale after confirming efficacy thereof in various types of cancer. However, overall response rates (ORRs) thereof are about 30 % on average, and there are still many problems to overcome. To address this issue, interleukin is considered an important agent for co-administration or as a dual-action partner with immune checkpoint inhibitors to enhance efficacy.

Among them, interleukin 2 (IL2), as a globular glycoprotein having a protein size of 15.5 kDa and consisting of 133 amino acids, plays a central role in lymphocyte production, survival, and homeostasis. IL2 is biosynthesized mainly by activated T cells, particularly by CD4+ helper T cells, to promote proliferation and differentiation of T cells, and stimulate production of cytotoxic T lymphocytes (CTLs) and production and proliferation of natural killer cells (NK cells). Therefore, IL2 may increase lymphocyte populations and increase the function of immune cell in the living body, and currently, therapy using IL2 has been approved and used for patients with metastatic renal cell carcinoma and malignant melanoma.

Lymphocyte activation of IL2 is mediated by action thereof in binding to a combination of three different IL2 receptors (IL2Rs) called IL2 receptor α (IL2Rα; CD25), IL2 receptor β (IL2Rβ; CD122), and common cytokine receptor γ (IL2R γ; CD132). Distribution of each subunit receptor varies according to cells and binding ability to IL2 also considerably varies according to each receptor. High-affinity IL2R consists of a trimer of three subunits (α, β, and γ), and a dimeric IL2 receptor consisting of the subunits βand γ is referred to as intermediate-affinity IL2R. Although the dimeric intermediate-affinity IL2R binds to IL2 with an affinity about 100 times lower than that of a trimeric high-affinity receptor, the dimeric and trimeric IL2Rs may transmit signals when bound to IL2. Thus, the subunit α, CD25, is not essential for IL2 signaling. While the subunit α imparts high affinity to a receptor thereof, subunit β and subunit γ are important for signal transduction. A trimeric IL2R including CD25 is expressed by regulatory T cells and endothelial cells. While the trimeric IL2R is temporarily induced by common activated T cells, the T cells express only the dimeric IL2R during a rest phase (Nature Review Immunology. 2012 12:180-190).

Regulatory T cells, as a sub-population of T cells that regulate the immune system, maintain immune-tolerance to self-antigens, are related to autoimmune disease, and interfere with the cancer treatment effects generally by inhibiting or downregulating activation induction and proliferation of effector T cells. Regulatory T cells by continuously express the highest levels of CD25, and thus have a higher binding affinity for IL2 than effector T cells, and is therefore emerging as a problem that reduces the efficacy of cancer treatment using IL2.

Regarding IL2 immunotherapy, side effects are caused by recombinant human IL2 treatment. Patients receiving high-dose IL2 treatments frequently experience systemic side effects such as severe cardiovascular, pulmonary, renal, hepatic, gastrointestinal, neurological, cutaneous, and hematological disorders, which require intensive monitoring and hospitalization. The major causes of these side effects may be explained by the development of so-called vascular (or capillary) leak syndrome (VLS), a pathological increase in vascular permeability leading to fluid extravasation in multiple organs (causing e.g. pulmonary and cutaneous edema and liver cell damage) and intravascular fluid depletion (causing a drop in blood pressure and compensatory increase in heart rate). There is no treatment of VLS other than withdrawal of IL2. Although low-dose IL2 administration has been tested in patients to avoid VLS, inappropriate therapeutic results of decreasing efficacy of cancer treatment have occurred. VLS was believed to be caused by the release of inflammatory cytokines, such as tumor necrosis factor (TNF)-α, from IL2-activated NK cells; however, it has recently been proven that IL2-induced pulmonary edema resulted from direct binding of IL2 to lung endothelial cells, which expressed low to intermediate levels of trimeric IL2R (International Immunology, 2006 vol. 18, no. 10 :1461-1471).

Therefore, despite the potential of IL2 as an immunotherapy for cancers, in order to reduce toxicity and side effects and increase therapeutic efficacy, there is a need to develop methods of treating cancer with increased efficacy while minimizing side effects caused by IL2, by using an IL2 variant that selectively activates effector T cells, a fragment crystallizable (Fc) region, an immune checkpoint and an antibodies targeting cancer cell specific antigens.

### Disclosure of Invention

### Technical Problem

An aspect provides a protein including an IL2 variant.

Another aspect provides a protein complex including an IL2 variant, a first polypeptide including a first CH3 antibody constant region and a second polypeptide including a second CH3 antibody constant region; and an antibody against an immune checkpoint inhibitor or an antigen-binding fragment thereof or an antibody against a tumor associated antigen or an antigen-binding fragment thereof.

Another aspect provides a method of preparing the protein or the protein complex.

Another aspect provides a pharmaceutical composition for preventing or treating cancer including the protein or protein complex as an active ingredient.

Another aspect provides a method of treating cancer including administering the protein or the protein complex.

Another aspect provides a use of the protein or protein complex for preparing an anticancer drug.

### Solution to Problem

An aspect provides a protein including an IL2 variant wherein the IL2 variant includes one or more amino acids selected from the group consisting of glutamic acid (E), alanine (A), lysine (K), and serine (S) at one or more positions selected from the group consisting of positions 35, 38, 42, and 125.

The IL2 variant may be a wild-type IL2 including amino acids of SEQ ID NO: 16 in which one or more amino acids are substituted. The IL2 variant may be one in which 1, 2, 3, 4, 5, 6, 7, 8, 9, or 10 or more amino acids are substituted as long as the activity of IL2 is maintained. For example, the IL2 variant may be a wild-type IL2 including amino acids of SEQ ID NO: 16 in which one or more positions selected from the group consisting of positions 18, 19, 35, 38, 42, 125, and 126 are substituted.

Specifically, the IL2 variant may include one or more amino acids selected from the group consisting of methionine (M), arginine (R), alanine (A), leucine (L), serine (S), phenylalanine (F), valine (V), isoleucine (I), glutamine (Q), tryptophan (W), asparagine (N), threonine (T), glutamic acid (E), and lysine (K) at one or more positions selected from the group consisting of positions 18, 19, 35, 38, 42, 125, and 126.

The IL2 variant may have increased or reduced binding ability to an IL2 receptor by substitution of amino acids. For example, the binding ability to the IL2Rα and/or IL2Rβγ may be reduced. Therefore, the IL2 variant may selectively activate effector T cells relative to regulatory T cells that inhibit immune activity by reducing binding ability to IL2R.

In an embodiment, the IL2 variant may include glutamic acid (E) at position 35, alanine (A) at position 38, lysine (K) at position 42, and serine (S) at position 125.

In another embodiment, the IL2 variant may include one or more amino acids selected from the group consisting of leucine (L), methionine (M), arginine (R), alanine (A), serine (S), phenylalanine (F), valine (V), isoleucine (I), glutamine (Q), tryptophan (W), asparagine (N), and threonine (T) at one or more positions selected from the group consisting of positions 18 and 19.

In another embodiment, the IL2 variant may further include one or more amino acids selected from the group consisting of threonine (T) and isoleucine (I) at position 126.

For example, the IL2 variant may include:
an amino acid selected from the group consisting of methionine (M), arginine (R), alanine (A), leucine (L), serine (S), phenylalanine (F), valine (V), isoleucine (I), and glutamine (Q) at position 18;
an amino acid selected from the group consisting of serine (S), leucine (L), tryptophan (W), asparagine (N), isoleucine (I), threonine (T), alanine (A), methionine (M), and phenylalanine (F) at position19;
glutamic acid (E) at position 35;
alanine (A) at position 38;
lysine (K) at position 42;
serine (S) at position 125; and
an amino acid selected from the group consisting of threonine (T) and isoleucine (I) at position 126.

Specifically, the IL2 variant may include one selected from the group consisting of:
glutamic acid (E) at position 35, alanine (A) at position 38, lysine (K) at position 42, and serine (S) at position 125;
methionine (M) at position 18, serine (S) at position 19, glutamic acid (E) at position 35, alanine (A) at position 38, lysine (K) at position 42, and serine (S) at position 125;
alanine (A) at position 18, serine (S) at position 19, glutamic acid (E) at position 35, alanine (A) at position 38, lysine (K) at position 42, and serine (S) at position 125;
arginine (R) at position 18, serine (S) at position 19, glutamic acid (E) at position 35, alanine (A) at position 38, lysine (K) at position 42, and serine (S) at position 125;
methionine (M) at position 18, leucine (L) at position 19, glutamic acid (E) at position 35, alanine (A) at position 38, lysine (K) at position 42, and serine (S) at position 125;
leucine (L) at position 18, serine (S) at position 19, glutamic acid (E) at position 35, alanine (A) at position 38, lysine (K) at position 42, and serine (S) at position 125;
serine (S) at position 18, tryptophan (W) at position 19, glutamic acid (E) at position 35, alanine (A) at position 38, lysine (K) at position 42, and serine (S) at position 125;
phenylalanine (F) at position 18, asparagine (N) at position 19, glutamic acid (E) at position 35, alanine (A) at position 38, lysine (K) at position 42, and serine (S) at position 125;
valine (V) at position 18, isoleucine (I) at position 19, glutamic acid (E) at position 35, alanine (A) at position 38, lysine (K) at position 42, and serine (S) at position 125;
isoleucine (I) at position 18, threonine (T) at position 19, glutamic acid (E) at position 35, alanine (A) at position 38, lysine (K) at position 42, and serine (S) at position 125;
glutamine (Q) at position 18, alanine (A) at position 19, glutamic acid (E) at position 35, alanine (A) at position 38, lysine (K) at position 42, and serine (S) at position 125;
methionine (M) at position 18, methionine (M) at position 19, glutamic acid (E) at position 35, alanine (A) at position 38, lysine (K) at position 42, and serine (S) at position 125;
methionine (M) at position 18, phenylalanine (F) at position 19, glutamic acid (E) at position 35, alanine (A) at position 38, lysine (K) at position 42, and serine (S) at position 125;
glutamic acid (E) at position 35, alanine (A) at position 38, lysine (K) at position 42, serine (S) at position 125, and threonine (T) at position 126; and
glutamic acid (E) at position 35, alanine (A) at position 38, lysine (K) at position 42, serine (S) at position 125, and isoleucine (I) at position 126.

In another embodiment, the IL2 variant may include amino acids selected from the group consisting of SEQ ID NOS: 1 to 15. In addition, the IL2 variant may include a polynucleotide encoding the amino acids. Specifically, the polynucleotide may be selected from the group consisting of SEQ ID NOS: 56 to 70.

In addition, the protein may include an Fc region linked by a linker or carrier. For example, the linker may include a copolymer such as 1 to 50 amino acids, albumin or fragments thereof, and polyethyleneglycol.

In an embodiment, the linker may include an amino acid sequence of SEQ ID NO: 18.

In another embodiment, the Fc region may include one or more amino acid sequences selected from the group consisting of SEQ ID NOS: 19 to 21; SEQ ID NOS: 22 to 24; SEQ ID NOS: 25 to 27; SEQ ID NOS: 28 to 30; SEQ ID NOS: 31 to 33; SEQ ID NOS: 34 to 36; SEQ ID NOS: 37 to 39; SEQ ID NOS: 40 to 42; SEQ ID NOS: 43 to 45; and SEQ ID NOS: 46 to 48.

In addition, the protein may further include an antibody against an immune checkpoint inhibitor or an antigen-binding fragment thereof. The immune checkpoint inhibitor may be, for example, PD-L1, PD-1, LAG3, VISTA, BTLA, TIM3, TIGIT, or CTLA-4.

In addition, the protein may further include an antibody against a tumor associated antigen or an antigen-binding fragment thereof. The tumor associated antigen may be, for example, PD-L1, EGFR, HER-2, B7H3, GPC3, CEA, TROP, or PSMA.

Therefore, another aspect provides a protein complex including: an IL2 variant; and an Fc region.

Also, another aspect provides a protein complex including: an IL2 variant; and an antibody against an immune checkpoint inhibitor or an antigen-binding fragment thereof.

Also, another aspect provides a protein complex including: an IL2 variant; and an antibody against a tumor associated antigen or an antigen-binding fragment thereof.

As used herein, the term "protein complex", as a combination of two or more associated polypeptides, refers to an artificial recombinant protein expressed after linking genes of one or more different proteins to a protein and expressing the genes and may be used interchangeably with "fusion protein". By linking two or more proteins, the protein complex may be expected to have a synergistic effect on functions thereof. Therefore, the protein complex, as a conjugate including an Fc region or a protein complex or fusion protein, may selectively increase the activity of effector T cells, relative to regulatory T cells, by substituting certain amino acids of IL2. In addition, because the protein complex may effectively induce the death of cancer cells by specifically being distributed in a tumor microenvironment, inhibiting immune checkpoints, and activating immune cells, a therapeutic agent with reduced side effects and maximized anticancer activity compared to existing therapeutic agents may be provided.

Another aspect provides a protein complex including a first polypeptide including a first CH3 antibody constant region and a second polypeptide including a second CH3 antibody constant region, wherein the first polypeptide and the second polypeptide form a heterodimer, an N-terminus of one or more the first or second polypeptide includes an antibody against an immune checkpoint inhibitor or an antigen-binding fragment thereof, or an antibody against a tumor associated antigen or an antigen-binding fragment thereof, and one or more of the N-terminus or the C-terminus of the first or second polypeptide include an IL2 variant.

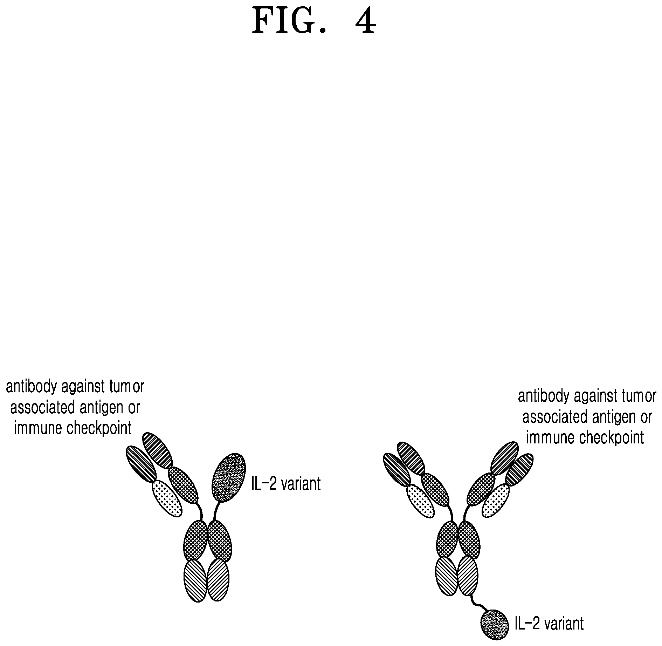

FIG. 4 shows a structure of a protein complex including an IL2 variant according to an embodiment.

Referring to FIG. 4, the protein complex may include an antibody against an immune checkpoint inhibitor or an antigen-binding fragment thereof, or an antibody against a tumor associated antigen or an antigen-binding fragment thereof at the N-terminus of the first polypeptide including the first CH3 antibody constant region, and an IL2 variant at the N-terminus of the second polypeptide including the second CH3 antibody constant region.

In addition, the protein complex may include an antibody against an immune checkpoint inhibitor or an antigen-binding fragment thereof, or an antibody against a tumor associated antigen or an antigen-binding fragment thereof at the N-terminus of the first polypeptide including the first CH3 antibody constant region and the second polypeptide including the second CH3 antibody constant region, and an IL2 variant at the C-terminus of the first or second polypeptide.

Detailed descriptions of the IL2 variant, the immune checkpoint inhibitor, and the tumor associated antigen are as given above. In the protein complex, the binding ability between the antibody against an immune checkpoint inhibitor or an antigen-binding fragment thereof and cancer cells or T cells is superior to the binding ability between T cells and IL2 receptors. Therefore, by primarily binding to cancer cells or T cells surrounding cancer cells, the antibody against an immune checkpoint inhibitor or an antigen-binding fragment thereof may reduce side effects caused by binding to IL2 receptors that may induce activation of systemic immune cells. In addition, the antibody against a tumor associated antigen or an antigen-binding fragment thereof not only specifically binds to cancer cells, but also have superior binding ability to cancer cells to the binding ability to an IL2 receptor-binding protein. Therefore, the antibody against a tumor associated antigen or an antigen-binding fragment thereof primarily binds to cancer cells to reduce side effects caused by binding to IL2 receptors that may induce activation of systemic immune cells.

The antibodies against an immune checkpoint inhibitor and/or a tumor associated antigen or antigen-binding fragments thereof may be, for example, antibody, antigen-binding fragment (Fab), single chain variable fragment (scFv), or nanobody.

As used herein, the term "antibody" is used interchangeably with the term "immunoglobulin (Ig)". A complete antibody has a structure having two full-length light chains and two full-length heavy chains, and each light chain is bound to a heavy chain by a disulfide bond (SS-bond). The light chain has two types, λ and κ, and consists of approximately 211 to 217 amino acids. There is only one type of a light chain in each human antibody. The light chain consists of a constant region and a variable region continuously connected. The heavy chain has five (γ, δ, α, µ, and ε) types, and the heavy chain determines the type of the antibody. α and γ consist of 450 amino acids, and µ and ε consist of 550 amino acids. The heavy chain has two regions, a variable region and a constant region. The variable region refers to a region to which an antigen binds in an antibody. The variable region may include a complementarity determining region (CDR) conferring antigen-binding specificity.

The antibody may include an antigen-binding fragment (Fab) region that binds to an antigen and a fragment crystallizable (Fc) region that binds to a cell surface receptor. When cleaved with papain, the complete antibody may be cleaved into two Fab regions and one Fc region. The Fab region may be one, in which a polypeptide including a heavy chain variable region (VH) domain and a heavy chain constant region 1 (CH1) domain is linked to a polypeptide including a light chain variable region (VL) domain and a light chain constant region (CL) domain, by a disulfide bond. The Fc region may be one in which two polypeptides including a heavy chain constant region 2 (CH2) domain and a constant region 3 (CH3) domain are linked. The Fc region may form a hinge region. The CH3 antibody constant region refers to the heavy chain constant region 3 domain of an antibody.

In general, the effector function of an antibody causes antibody dependent cell cytotoxicity (ADCC) or complement dependent cytotoxicity (CDC) as the Fc receptor binds to an Fcγ receptor as a receptor thereof or C1q as a complement component. Because immune cells have many Fcγ receptors, undesirable death may be induced, and thus it is preferable to remove the effector function. Therefore, the CH3 antibody constant region may be one modified to have high stability by reducing the binding ability to Fcγ receptors or removing or reducing the effector function. In addition, the CH3 antibody constant region may be one modified to have reduced antibody-dependent cellular cytotoxicity (ADCC).

In an embodiment, the Fc region may include tryptophan (W) at position 366 of the first CH3 antibody constant region, and serine (S) at position 366, alanine (A) at position 368, and valine (V) at position 407 of the second CH3 antibody constant region; and glycine (G) or phenylalanine (F) at position 351 of the second CH3 antibody constant region.

In addition, the Fc region may include serine (S) at position 366, alanine (A) at position 368, and valine (V) at position 407 of the first CH3 antibody constant region, and tryptophan (W) at position 366 of the second CH3 antibody constant region; and glycine (G) or phenylalanine (F) at position 351 of the first CH3 antibody constant region.

In addition, the Fc region may include tryptophan (W) at position 366 of the first CH3 antibody constant region, and glycine (G) at position 351, serine (S) at position 366, alanine (A) at position 368, and valine (V) at position 407 of the second CH3 antibody constant region; and phenylalanine (F) or tryptophan (W) at position 351 of the first CH3 antibody constant region.

In addition, the Fc region may include glycine (G) at position 351, serine (S) at position 366, alanine (A) at position 368, and valine (V) at position 407 of the first CH3 antibody constant region, and tryptophan (W) at position 366 of the second CH3 antibody constant region; and phenylalanine (F) or tryptophan (W) at position 351 of the second CH3 antibody constant region.

In another embodiment, the first CH antibody constant region and the second CH antibody constant region may further include one or more amino acids selected from the group consisting of alanine (A), glycine (G), glutamine (Q), phenylalanine (F), glutamic acid (E), and serine (S) at one or more positions selected from the group consisting of positions 234, 235, 329, 297, 331, and 265. Specifically, the CH antibody constant region may be a CH2 antibody constant region.

For example, the first CH2 antibody constant region and the second CH2 antibody constant region may be one in which leucine (L) at positions 234 and 235 is further substituted with alanine (A) (L234A/L235A).

In addition, the first CH2 antibody constant region and the second CH2 antibody constant region may be one in which leucine (L) at positions 234 and 235 is further substituted with alanine (A), and proline (P) at position 329 is further substituted with glycine (G) (L234A/L235A/P329G).

In addition, the first CH2 antibody constant region and the second CH2 antibody constant region may be one in which asparagine (N) at position 297 is further substituted with alanine (A), glutamine (Q), or glycine (G) (N297A, N297Qor N297G).

In addition, the first CH2 antibody constant region and the second CH2 antibody constant region may be one in which leucine (L) at positions 234 and 235 is substituted with phenylalanine (F) and glutamic acid (E), and proline (P) at position 331 is further substituted with serine (S) (L234F/L235E/P331S).

In addition, the first CH2 antibody constant region and the second CH2 antibody constant region may be one in which leucine (L) at positions 234 and 235 is substituted with phenylalanine (F) and glutamic acid (E), and aspartic acid (D) at position 265 is further substituted with alanine (A) (L234F/L235E/D265A).

In addition, in another embodiment, the Fc region may include an amino acid sequence selected from the group consisting of SEQ ID NOS: 19 to 21; SEQ ID NOS: 22 to 24; SEQ ID NOS: 25 to 27; SEQ ID NOS: 28 to 30; SEQ ID NOS: 31 to 33; SEQ ID NOS: 34 to 36; SEQ ID NOS: 37 to 39; SEQ ID NOS: 40 to 42; SEQ ID NOS: 43 to 45; and SEQ ID NOS: 46 to 48.

In addition, in another embodiment, the first polypeptide or the second polypeptide; and the IL2 variant may be linked by a linker or carrier. The linker may have an amino acid sequence of (GGGGS)n, and the "n" may be a natural number from 1 to 10. Specifically, the linker may include an amino acid sequence of SEQ ID NO: 18.

The first polypeptide and the second polypeptide may form a Fc region of an antibody.

The heterodimer refers to a combination of two polypeptides having different sequences, numbers, or types of amino acid residues. The protein complex may be a protein complex formed by combining of two polypeptides that specifically bind to different targets.

The protein complex may be an antibody or an antigen-binding fragment thereof, a conjugate of a receptor and an agonist, a conjugate of a receptor and an antagonist, a conjugate of a receptor and a ligand, or a conjugate of a ligand and a decoy receptor. In addition, the protein complex may include any one selected from the group consisting of an antigen-binding fragment (Fab), a single chain variable fragment (scFv), an extracellular domain of a membrane receptor, an agonist, an antagonist, a ligand, a decoy receptor, a cytokine, a coagulation factor, and an affinity tag.

The antibody may be, for example, IgA, IgD, IgE, IgG, or IgM. The antibody may be a monoclonal antibody or a polyclonal antibody. The antibody may be an animal-derived antibody, a mouse-human chimeric antibody, a humanized antibody, or a human antibody.

As used herein, the term "antigen-binding fragment" refers to a fragment of an entire immunoglobulin structure, indicating a portion of a polypeptide including a part to which an antigen is able to bind. For example, the antigen binding fragment may be scFv, (scFv)₂, Fv, Fab, Fab', Fv F(ab')₂, or a combination thereof.

As used herein, the term "receptor" refers to a substance receiving or transmitting a signal that may be transmitted to a biological system. The receptor may be a protein receptor. The receptor may bind to an agonist, an antagonist, a ligand, or a cytokine. The agonist may be a substance that binds to a receptor to activate the receptor, thereby inducing a biological response. The antagonist may be a substance that binds to a receptor to inhibit the receptor, thereby inhibiting a biological response. The ligand may be a substance that binds to a receptor. The ligand may bind to a decoy receptor. The decoy receptor refers to a receptor that specifically binds to a ligand, thereby inhibiting signal transduction by an actual receptor. The cytokine refers to a small protein that acts on cell signaling, and regulation and maintenance of inflammatory processes.

The protein complex may be modified. For example, the protein complex may be modified by conjugation or binding, glycosylation, tag attachment, or a combination thereof. The antibody may be conjugated with other drugs such as anticancer drugs. For example, the protein complex may be one combined with a horseradish peroxidase (HRP), an alkaline phosphatase, hapten, biotin, streptavidin, a fluorescent material, a radioactive material, quantum dots, polyethylene glycol (PEG), a histidine tag, or a combination thereof. The fluorescent material may be Alexa Fluor^{®}532, Alexa Fluor^{®}546, Alexa Fluor^{®}568, Alexa Fluor^{®}680, Alexa Fluor^{®}750, Alexa Fluor^{®}790, or Alexa Fluor^{®}350.

The positions of the amino acids of the Fc region or CH2, and CH3 region are as described in the Kabat EU index ('Kabat et al., Sequences of Proteins of Immunological Interest, 5th Ed. Public Health Service, National Institutes of Health, Bethesda, MD. (1991)'). The positions of the amino acids of the CH3 domain and the corresponding amino acid types are based on human IgG1.

Another aspect provides a polynucleotide encoding a protein according to an aspect or a protein complex according to another aspect.

Detailed descriptions of the protein or protein complex are as given above.

In an embodiment, the polynucleotide may include one or more polynucleotides selected from the group consisting of SEQ ID NOS: 56 to 70; and one or more polynucleotides selected from the group consisting of SEQ ID NOS: 74 to 76.

In another embodiment, the polynucleotide may include one or more polynucleotides selected from the group consisting of SEQ ID NOS: 56 to 70; a polynucleotide including SEQ ID NO: 73; and one or more polynucleotides selected from the group consisting of SEQ ID NOS: 74 to 76.

In another embodiment, the polynucleotide may include one or more polynucleotides selected from the group consisting of SEQ ID NOS: 56 to 70; a polynucleotide including SEQ ID NO: 73; one or more polynucleotides selected from the group consisting of SEQ ID NOS: 74 to 76; and one or more polynucleotides selected from the group consisting of SEQ ID NOS: 77 to 82.

Another aspect provides a method of preparing a protein or protein complex, the method including expressing the protein or protein complex by transforming cells with an expression vector encoding the protein according to an aspect or the protein complex according to another aspect. In addition, another aspect provides a protein or protein complex including an IL2 variant prepared by the method.

Detailed descriptions of the protein or protein complex are as given above.

Expression vector, as an expression vector capable of expressing a target protein in appropriate host cells, refers to a vector including essential regulatory elements operably linked so as to express an inserted nucleic acid sequence. The "operably linked" means that a nucleic acid expression regulatory sequence and a nucleic acid encoding a target protein are functionally linked to perform a general function. The expression vector may include a polynucleotide encoding protein the complex. The expression vector may include a regulatory region necessary for gene expression, such as an enhancer, a promoter, and a poly(A) sequence.

The cells may be cancer cells. The cells may be in vitro cells. The cells may be bacteria, yeasts, plant cells, or mammalian cells. The bacteria may be E. *coli.* The mammalian cells refer to cells derived from mice, rats, rabbits, dogs, cats, sheep, cows, horses, monkeys, chimpanzees, or humans. The cells may be cell lines. The cells may be, for example, selected from the group consisting of Chinese hamster ovary (CHO) cells, human embryonic kidney (HEK) cells, baby hamster kidney (BHK) cells, NS0 cells, PER.C6 cells, HeLa cells, Madin-Darby Canine Kidney (MDCK) cells, SP2/0 mouse myeloma cells, COS-7, and YB2/0 rat myeloma cells. The CHO cells may be CHO DG44, CHO-K1, CHO-S, GS-CHO, or CHO DUKX (DXB11) cells. The HEK cells may be HEK 293 cells.

"Transformation" refers to a method of inserting a specific nucleic acid fragment into the genome of a cell so that the inserted nucleic acid is expressed.

In an embodiment, the method may include co-transfecting cells with an expression vector encoding the first and/or second polypeptide including an antibody against an immune checkpoint inhibitor or an antigen-binding fragment thereof, or an antibody against a tumor associated antigen or an antigen-binding fragment thereof at the N-terminus of one or more of the first or second polypeptide, and an expression vector encoding the first or second polypeptide including an IL2 variant at the N-terminus or C-terminus. In addition, the method may include transforming two or more types of cells respectively with an expression vector encoding the first polypeptide and/or second polypeptide including an antibody against an immune checkpoint inhibitor or an antigen-binding fragment thereof, or an antibody against a tumor associated antigen or an antigen-binding fragment thereof at the N-terminus thereof, and an expression vector encoding the first or second polypeptide including an IL2 at the N-terminus or C-terminus.

The cells may be cultured in a cell culture medium. The cell culture medium refers to a solution containing nutrients necessary for culturing cells. The medium includes a commercialized or prepared medium used for culturing cells. The cell culture medium may contain an antibiotic. The cell culture medium may include G418 (geneticin), Puromycin, Blasticidin, Zeocin, or a combination thereof. The cell culture medium may include a chemically defined medium.

The cells may be cultured under conditions that allow survival or proliferation of the cells. Conditions allowing survival or proliferation of the cells may vary depending on the type of the cells. The cells may be cultured at about 25 °C to about 42 °C, about 25 °C to about 40 °C, about 30 °C to about 40 °C, about 30 °C to about 37 °C, or about 37 °C. The cells may be cultured in the presence of air having about 1% CO₂ to about 10% CO₂ or about 5% CO₂ to about 10% CO₂. The cells may be cultured in a medium of about pH 6 to about pH 8, about pH 6.2 to about pH 7.8, about pH 6.4 to about pH 7.6, about pH 6.6 to about pH 7.4, or about pH 6.8 to about pH 7.2. The cells may be cultured in a condition of dissolved oxygen of about 10% to about 80%, about 15% to about 70%, or about 20% to about 60%.

The culture may vary depending on the type of the cells. The culture may use a known method. The culture may be performed on a plate, flask, or the like. The culture may be performed by attaching the cells to a substrate or floating the cells in a culture medium. The culture may be a subculture, a batch culture, a fed-batch culture, a perfusion culture, or a combination thereof. During the culture, the cell culture medium may be periodically exchanged with a fresh medium. The cells may be cultured for about 1 day or more, about 2 days or more, about 3 days or more, about 4 days or more, about 5 days or more, about 6 days or more, about 1 week or more, about 10 days or more, about 2 weeks or more, about 3 weeks or more, about 1 month or more, from about 1 day to about 1 month, about 1 day to about 3 weeks, about 1 day to about 2 weeks, about 2 days to about 2 weeks, about 3 days to about 2 weeks, about 4 days to about 2 weeks, about 5 days to about 2 weeks, about 6 days to about 2 weeks, or about 1 week to about 2 weeks.

Obtaining a protein complex from the cells or the cell culture medium may be included.

The cell culture medium may be a culture medium without the cells.

In the case where the cells are co-transformed with the expression vectors, the protein complex of the first polypeptide including an antibody against an immune checkpoint inhibitor or an antigen-binding fragment thereof, or an antibody against a tumor associated antigen or an antigen-binding fragment thereof at the N-terminus, and the second polypeptide including an IL2 variant at the N-terminus or C-terminus may be obtained from the cells or cell culture media. In the case where two types of cells are transformed respectively with the expression vector encoding the first polypeptide including an antibody against an immune checkpoint inhibitor or an antigen-binding fragment thereof, or an antibody against a tumor associated antigen or an antigen-binding fragment thereof at the N-terminus, and the expression vector encoding the second polypeptide including an IL2 variant at the N-terminus or C-terminus, the first polypeptide including the antibody against an immune checkpoint inhibitor or an antigen-binding fragment thereof, or the antibody against a tumor associated antigen or an antigen-binding fragment thereof at the N-terminus; and the second polypeptide including the IL2 variant at the N-terminus or C-terminus may be obtained from the cells or cell culture media.

The obtaining of the protein complex may include forming the protein complex by incubating the obtained first polypeptide including the antibody against an immune checkpoint inhibitor or an antigen-binding fragment thereof, or the antibody against a tumor associated antigen or an antigen-binding fragment thereof at the N-terminus, and the obtained second polypeptide including the IL2 variant at the N-terminus or C-terminus. The incubation may be performed under a reducing condition. The reducing condition may be in the presence of 2-mercaptoethanol (2-ME), dithiothreitol (DTT), or a combination thereof.

The obtaining the protein complex may include purifying the protein complex. The purification may be performed by filtration, centrifugation, chromatography, dialysis, immunoprecipitation, or a combination thereof.

Another aspect provides a pharmaceutical composition for preventing or treating cancer including a protein according to an aspect or a protein complex according to another aspect. Another aspect provides a use of a protein according to an aspect or a protein complex according to another aspect for preparing a prophylactic or therapeutic agent of cancer.

Detailed descriptions of the protein or the protein complex are as given above.

The cancer may be a solid cancer or a non-solid cancer. Solid cancer refers to cancerous tumors that occurred in organs such as liver, lung, breast, skin, etc. Non-solid cancers are cancers that occurred in the blood and are also called blood cancers. The cancer may be carcinoma, sarcoma, hematopoietic cell-derived cancer, germ cell tumor, or blastoma. The cancer may be selected from the group consisting of breast cancer, skin cancer, head and neck cancer, pancreatic cancer, lung cancer, colon cancer, colorectal cancer, stomach cancer, ovarian cancer, prostate cancer, bladder cancer, urethral cancer, liver cancer, kidney cancer, clear cell sarcoma, melanoma, cerebrospinal tumor, brain cancer, thymoma, mesothelioma, esophageal cancer, biliary tract cancer, testicular cancer, germ cell tumor, thyroid cancer, parathyroid cancer, cervical cancer, endometrial cancer, lymphoma, myelodysplastic syndromes (MDS), myelofibrosis, acute leukemia, chronic leukemia, multiple myeloma, Hodgkin's disease, endocrine cancer, and sarcoma.

The "prevention" refers to any action that inhibits a disease or delays the onset of a disease by administration of the pharmaceutical composition. The term "treatment" refers to any action that ameliorates or beneficially changes the symptoms of a disease by administration of the pharmaceutical composition.

The pharmaceutical composition may include a pharmaceutically acceptable carrier. The carrier is used to include excipients, diluents, or adjuvants. The carrier may be, for example, selected from the group consisting of lactose, dextrose, sucrose, sorbitol, mannitol, xylitol, erythritol, maltitol, starch, acacia rubber, alginate, gelatin, calcium phosphate, calcium silicate, cellulose, methyl cellulose, polyvinylpyrrolidone, water, physiological saline, buffers such as PBS, methylhydroxy benzoate, propylhydroxy benzoate, talc, magnesium stearate, and mineral oil. The composition may include a filler, an anti-agglomeration agent, a lubricant, a wetting agent, a flavoring agent, an emulsifying agent, a preservative, or a combination thereof.

The pharmaceutical composition may be prepared in any formulation according to a method in the art. The composition may be formulated, for example, as a formulation for oral administration (e.g., powder, tablet, capsule, syrup, pill, or granule), or a formulation for parenteral administration (e.g., injection). In addition, the composition may be prepared as a systemic formulation, or as a local formulation.

The pharmaceutical composition may further include other anticancer agents. The anticancer agent may be cetuximab, panitumumab, erlotinib, gefitinib, trastuzumab, T-DM1, perjeta, lapatinib, paclitaxel, taxol, tamoxifen, cisplatin, or a combination thereof. The pharmaceutical composition may be a single composition or separate compositions. For example, the composition of an antibody or an antigen-binding fragment thereof may be a composition for parenteral administration, and the anticancer agent may be a composition for oral administration.

The pharmaceutical composition may include the protein complex in an effective amount. The term "effective amount" means an amount sufficient to exhibit preventive or therapeutic effects when administered to a subject in need of prevention or treatment of a disease. The effective amount may be appropriately selected by those skilled in the art depending on the cell or the subject. The effective amount may be determined based on the severity of disease, age, body weight, health status, and gender of the patient, patient's sensitivity to the drug, administration time, route of administration, and excretion rate, duration of treatment, drug used in combination or simultaneously with the composition used, and other factors well known in the medical field. The effective amount may be about 0.5 µg to about 2 g, about 1 µg to about 1 g, about 10 µg to about 500 mg, about 100 µg to about 100 mg, or about 1 mg to about 50 mg per the pharmaceutical composition.

The dosage of the pharmaceutical composition may be, for example, in the range of from about 0.001 mg/kg to about 100 mg/kg, from about 0.01 mg/kg to about 10 mg/kg, or from about 0.1 mg/kg to about 1 mg/kg, for an adult. The administration may be performed once a day, multiple times a day, once a week, once every 2 weeks, once every 3 weeks, or once every 4 weeks to once a year.

Another aspect provides a method of preventing or treating cancer, the method including administering a protein according to an aspect or a protein complex according to another aspect to a cell or a subject.

Detailed descriptions of the protein, protein complex, cell, cancer, prevention, or treatment are as given above.

The subject may be a mammal, such as, human, cow, horse, pig, dog, sheep, goat, or cat. The subject may be a subject that has cancer or is likely to have cancer.

The method may further include administering a second active ingredient to the subject. The second active ingredient may be an active ingredient for preventing or treating cancer. The active ingredient may be administered simultaneously, separately, or sequentially with the protein complex.

The protein or protein complex may be administered, for example, directly into a subject by any method, such as, oral, intravenous, intramuscular, transdermal, mucosal, intranasal, intratracheal, or subcutaneous administration. The protein complex may be administered systemically or locally, alone or in combination with other pharmaceutically active compounds.

The preferred dosage of the protein or protein complex may vary depending upon the patient's condition and body weight, severity of the disease, formulation of the drug, route and duration of administration, etc., and may be appropriately selected by those skilled in the art. The dosage may be, for example, in the range of from about 0.001 mg/kg to about 100 mg/kg, from about 0.01 mg/kg to about 10 mg/kg, or from about 0.1 mg/kg to about 1 mg/kg for an adult. For the administration, the protein complex may be administered once a day, multiple times a day, once a week, once every 2 weeks, once every 3 weeks, or once every 4 weeks to once a year.

### Advantageous Effects of Invention

The protein according to an aspect may selectively increase the activity of effector T cells, relative to the regulatory T cells, by substituting a certain amino acid sequence of IL2. In addition, the pharmaceutical composition including the protein may reduce side effects and maximize anticancer activity compared to existing therapeutic agents, the pharmaceutical composition may be used to prevent or treat cancer.

### Brief Description of Drawings

FIG. 1A shows results of purifying a PD-L1 antibody-IL2 variant 2 protein complex according to an embodiment by using a Protein A affinity column purification method.
FIG. 1B shows results of purifying a PD-L1 antibody-IL2 variant 2 protein complex according to an embodiment by using a cation exchange resin column purification method.
FIG. 1C shows results of analyzing purity of a PD-L1 antibody-IL2 variant 2 protein complex according to an embodiment by SE-HPLC analysis.
FIG. 2A shows results of comparison of anticancer effects of a PD-L1 antibody-IL2 variant 2 protein complex according to an embodiment, positive controls (Avelumab, and co-administration of Avelumab and Aldesleukin), and a negative control, in colorectal cancer syngeneic mice.
FIG. 2B shows results of identifying cancer cell growth inhibition and complete remission activity of a PD-L1 antibody-IL2 variant 2 protein complex according to an embodiment in colorectal cancer syngeneic mice, per subject.
FIG. 2C shows results of identifying cancer cell growth inhibition and complete remission effect of a negative control and positive controls (Avelumab, and co-administration of Avelumab and Aldesleukin) on colorectal cancer syngeneic mice, per subject.
FIG. 3 shows results of identifying anticancer activity of a PD-L1 antibody-IL2 variant 4 protein complex according to an embodiment in colorectal cancer syngeneic mice.
FIG. 4 shows a structure of a protein complex including an IL2 variant according to an embodiment.
FIG. 5A shows results of purifying a mousePD-1 antibody-IL2 variant 14 protein complex (a+c) according to an embodiment by using a Protein A affinity column purification method.
FIG. 5B shows results of purifying a mousePD-1 antibody-IL2 variant 14 protein complex (a+c) according to an embodiment by using a hydrophobic interaction column purification method.
FIG. 5C shows results of analyzing purity of a mousePD-1 antibody-IL2 variant 14 protein complex (a+c) according to an embodiment.
FIG. 6A shows results of comparing anticancer activities of a mousePD-1 antibody-IL2 variant 14 protein complex (a+c) according to an embodiment, a negative control and a positive control, in a colorectal cancer syngeneic mouse model.
FIG. 6B shows results of comparing changes in body weight of a colorectal cancer syngeneic mouse model after administering a mousePD-1 antibody-IL2 variant 14 protein complex (a+c) according to an embodiment, a negative control, and a positive control to the colorectal cancer syngeneic mouse model.

### Mode for the Invention

Hereinafter, the disclosure will be described in more detail with reference to the following examples. However, the following examples are merely presented to exemplify the disclosure, and the scope of the disclosure is not limited thereto.

### [Examples]

### Example 1. Preparation of IL2 Variant Protein and Confirmation of Activity thereof

### 1-1. Cloning and Culture of IL2 Variant Protein

IL2 variants were prepared by substituting amino acids at some positions of an amino acid sequence (SEQ ID NO: 16) of a wild-type IL2. Specifically, after synthesizing a polynucleotide encoding an IL2 variant tagged with 6X histidine by using the Invitrogen GeneArt Gene Synthesis service of ThermoFisher Scientific Inc., an expression vector expressing the IL2 variant was prepared via insertion into Apall-Nhel enzyme site of a pcDNA3.1 expression vector. In the IL2 variants, substitution sites of each amino acid and substituted amino acids are as shown in Table 1 below. Then, a cell line was transfected with the expression vector by using an ExpiFectamine^{™} CHO Transfection kit (ThermoFisher). The transfected cells were cultured at 32 °C in a 5 % CO₂ incubator at 120 rpm for 12 days. After 12 days, the supernatant was separated and harvested, and then filtered through sterile filters to obtain the IL2 variant protein.

**[Table 1]**

| | 35 | 38 | 42 | 125 | 18 | 19 | 126 |
|---|---|---|---|---|---|---|---|
| wt-IL2 | K | R | F | C | L | L | Q |
| IL2 comparison group | E | | | S | | | |
| IL2 variant 1 | E | A | K | S | | | |
| IL2 variant 2 | E | A | K | S | M | S | |
| IL2 variant 3 | E | A | K | S | A | S | |
| IL2 variant 4 | E | A | K | S | R | S | |
| IL2 variant 5 | E | A | K | S | M | L | |
| IL2 variant 6 | E | A | K | S | L | S | |
| IL2 variant 7 | E | A | K | S | S | W | |
| IL2 variant 8 | E | A | K | S | F | N | |
| IL2 variant 9 | E | A | K | S | V | I | |
| IL2 variant 10 | E | A | K | S | I | T | |
| IL2 variant 11 | E | A | K | S | Q | A | |
| IL2 variant 12 | E | A | K | S | M | M | |
| IL2 variant 13 | E | A | K | S | M | F | |
| IL2 variant 14 | E | A | K | S | | | T |
| IL2 variant 15 | E | A | K | S | | | I |

### 1-2. Purification of IL2 Variant Protein

After purification by using His tagged on the IL2 variant protein obtained from the culture medium obtained in Example 1-1, a high-purity substance with a purity of 95 % or more was obtained.

First, the culture medium obtained in Example 1-1 was centrifuged to separate the medium from the cultured cells. Then, fine residues were removed from the IL2 variant protein contained in the separated medium using a 0.22 µm filter (Thermo Scientific Inc.). After the filtered medium was primarily purified by imidazole-affinity chromatography (Ni-SepFast, Biotoolomics Ltd.), an imidazole buffer component contained in the purified product was removed by desalination and concentration processes. Then, the content and purity of the final purified product were analyzed. Specifically, after adding the filtered medium to an Ni-SepFast column stabilized by a phosphate buffer saline (pH 7.4), nonspecifically unbound proteins were washed away by using the same buffer, and proteins specifically bound to the Ni-SepFast column were eluted using imidazole buffer solutions (0 M and 0.5 M, pH 7.4) by an imidazole concentration increasing gradient method. The obtained IL2 variant protein was stabilized by a desalination process using a phosphate buffer saline (pH 7.4), and purity of the purified IL2 variant protein was analyzed by using size exclusion HPLC (TSK-3000SWxL, 7.8 mm x 30 cm, Tosoh Inc.). In addition, molecular weights were identified by SDS-PAGE.

### 1-3. Identification of Activation of Human Immune cell by IL2 Variant Protein

Activation of human immune cells by the IL2 variant-protein complex purified in Example 1-2 was identified. Specifically, peripheral blood mononuclear cells (StemCell Inc.) were reacted with antibodies specifically fluorescence-labeling effector CD8+ T cells (CD3+ and CD8+) and Treg cells (CD4+ and CD25+) for 30 minutes at 4 °C in the dark, and antibodies that are not bound to the cells were removed by centrifugation. Then, the cells were treated with the IL2 variant 2, the IL2 variant 4, and the IL2 variant 14 purified in Example 1-2, followed by reaction for 20 minutes at 37 °C in the dark, and fixation using 1 mL of a fixation buffer (BD, U.S.) for 12 minutes. In this regard, Aldesleukin (Proleukin, Novartis, Switzerland) was used as a positive control and each drug was treated in a concentration of 0.01 to 8,000 nM. Subsequently, cytoplasm of the fixed peripheral blood mononuclear cells was treated with 1.5 mL of a Perm3 buffer (BD, U.S.) and reacted for 35 minutes at 4 °C to allow fluorescent labels to penetrate into the cytoplasm, and then phosphorylated STAT-5 and the Treg cell marker Foxp3+, were fluorescence labeled, followed by reaction for 30 minutes. Then, STAT-5 phosphorylation degrees of the IL2 variant protein-treated groups in the effector CD8+ T cells and Treg cells were compared by flow cytometry.

**[Table 2]**

| | STAT5 phosphorylation (EC₅₀, nM) | |
|---|---|---|
| | Treq cell | CD8+ T cell |
| Aldesleukin | 0.048 | 0.78 |
| IL2 variant 2 protein | 0.91 | 0.55 |
| IL2 variant 4 protein | 2.16 | 1.22 |
| IL2 variant 14 protein | 0.30 | 1.7 |

As a result, as shown in Table 2, the EC₅₀ value ratio of CD8+ T cell/regulatory T cell was 16.3 in the positive control (Aldesleukin), indicating that the activity of the Treg cells was induced more strongly than that of the CD8+ T cells.

On the contrary, the EC₅₀ value ratios of CD8+ T cell/regulatory T cell were 0.6, 0.56, and 5.6 in the IL2 variants 2, 4, and 14 proteins, respectively, indicating that the activity of the CD8+ T cells was more selectively activated in comparison with the positive control.

### Example 2. Preparation of IL2 Variant-Fc Protein Complex and Identification of Property

### 2-1. Cloning and Culture of IL2 Variant-Fc Protein Complex

Fc protein complexes including an IL2 variant were prepared. Specifically, after synthesizing a polynucleotide encoding an Fc region using the Invitrogen GeneArt Gene Synthesis service of ThermoFisher Scientific Inc., a first expression vector expressing the Fc region was prepared via insertion into AvrII-BstZ17I enzyme site of a pCHO1.0 expression vector. After synthesizing a polynucleotide encoding a complex including the IL2 variants 1, 2, 4, and 14, as well as a linker and an Fc region, by using the same service, a second expression vector expressing IL2 variant-Fc was prepared via insertion into Avrll-BstZ171 enzyme site of a pCHO1.0 expression vector. Subsequently, after mixing the first expression vector and the second expression vector in a ratio of 1:1 by using an ExpiFectamine^{™} CHO Transfection kit (ThermoFisher), ExpiCHO-S^{™} cell lines were transfected therewith. The transfected cells were cultured at 32 °C in a 8% CO₂ incubator at 125 rpm for 12 days. After 12 days, the supernatant of the culture medium was separated and harvested, and then filtered through sterile filters to obtain a protein complex including Fc and IL2 variant-Fc.

### 2-2. Purification of IL2 Variant-Fc Protein Complex

A high-purity substance was obtained by purifying the IL2 variant-Fc protein complex obtained by culturing the cell line prepared in Example 2-1.

First, the obtained culture medium obtained in Example 2-1 was centrifuged to separate the medium from the cultured cells. Then, fine residues were removed from the IL2 variant-Fc protein complex contained in the separated medium using a 0.22 µm filter (Thermo Scientific Inc.). The medium after a filtering process was initially purified by Protein A-affinity chromatography (MabSelect PrismA, Cytiva). Specifically, after adding the filtered medium to a Protein A column stabilized by a phosphate buffer saline (pH 7.4), nonspecifically unbound proteins were washed using the same buffer, proteins specifically bound to the Protein A column were eluted using two solutions containing 0.02 M citric acid (pH 5.0 and pH 3.5) according to a pH gradient, and the sample was neutralized to pH 7.2 by using 1 M Tris. Then, the obtained IL2 variant-Fc protein complexes were stabilized by a phosphate buffer saline (pH 7.4) and purity of the purified IL2 variant-Fc protein complex was analyzed by using size exclusion HPLC (TSK-3000SWxL, 7.8 mm x 30 cm, Tosoh Inc.).

### 2-3. Identification of Activation of Human Immune cellby IL2 Variant-Fc-Protein Complex

Activation of human immune cells by the IL2 variant-protein complex purified in Example 2-2 was identified. Specifically, peripheral blood mononuclear cells (StemCell Inc.) were reacted with antibodies specifically fluorescence-labeling effector CD8+ T cells (CD3+ and CD8+) and Treg cells (CD4+ and CD25+) for 30 minutes at 4 °C in the dark, and antibodies that are not bound to the cells were removed by centrifugation. Then, the cells were treated with the IL2 variant 1-Fc protein complex, the IL2 variant 2-Fc protein complex, the IL2 variant 4-Fc protein complex, and the IL2 variant 14-Fc protein complex purified in Example 2-2, followed by reaction for 20 minutes at 37 °C in the dark, and fixation using 1 mL of a fixation buffer (BD, U.S.) for 12 minutes. In this regard, Aldesleukin (Proleukin, Novartis, Switzerland) was used as a positive control and each drug was treated in a concentration of 0.01 to 8,000 nM. Subsequently, cytoplasm of the fixed peripheral blood mononuclear cells was treated with 1.5 mL of a Perm3 buffer (BD, U.S.) and reacted for 35 minutes at 4 °C to allow fluorescent labels to penetrate into the cytoplasm, and then phosphorylated STAT-5 and the Treg cell marker Foxp3+, were fluorescence labeled, followed by reaction for 30 minutes. Then, STAT-5 phosphorylation degrees of the IL2 variant-Fc protein complex-treated groups in the effector CD8+ T cells and Treg cells were compared by flow cytometry.

**[Table 3]**

| | STAT5 phosphorylation (EC₅₀, nM) | |
|---|---|---|
| | Treq cell | CD8+ T cell |
| Aldesleukin | 0.03 | 1 |
| IL2 variant 1-Fc protein complex | 2.91 | 2.31 |
| IL2 variant 2-Fc protein complex | 13.3 | 19.6 |
| IL2 variant 4-Fc protein complex | 24.3 | 35 |
| IL2 variant 14-Fc protein complex | 64.84 | 209.1 |

As a result, as shown in Table 3, the EC₅₀ value ratio of CD8+ T cell/regulatory T cell was 33.3 in the positive control (Aldesleukin), indicating that the activity of the Treg cells was induced more strongly than that of the CD8+ T cells.

On the contrary, the EC₅₀ value ratios of CD8+ T cell/regulatory T cell were 0.79 to 3.2 in the IL2 variant 1-Fc protein complex, the IL2 variant 2-Fc protein complex, the IL2 variant 4-Fc protein complex, and the IL2 variant 14-Fc protein complex, respectively, indicating that the CD8+ T cells were activated more strongly than the positive control.

That is, it was confirmed that the property of selectively activating CD8+ cells more than Treg may be maintained in the structure of IL2 variant according to an aspect even added with Fc.

### Example 3. Preparation of PD-L1 Antibody-IL2 Variant Protein Complex and Identification of Property

### 3-1. Cloning and Culture of PD-L1 Antibody-IL2 Variant Protein Complex

Protein complexes including a PD-L1 antibody and an IL2 variant were prepared. Specifically, a polynucleotide encoding a PD-L1 antibody heavy chain variable region-heavy chain constant region and a polynucleotide encoding a PD-L1 antibody light chain constant region-light chain variable region were synthesized, respectively, by using the same service used in Example 2-1. Then, a first expression vector expressing a PD-L1 antibody was prepared by inserting the polynucleotide encoding the PD-L1 antibody heavy chain variable region-heavy chain constant region and the polynucleotide encoding the PD-L1 antibody light chain constant region-light chain variable region respectively into AvrII-BstZ17I enzyme site and EcoRV-PacI enzyme site of a pCHO1.0 expression vector. After synthesizing a polynucleotide encoding a complex including one of the IL2 variants 1 to 15 prepared in Example 1, a linker, and an Fc region by using the same service, a second expression vector expressing the IL2 variant-Fc was prepared via insertion into AvrII-BstZ17I enzyme site of a pCHO1.0 expression vector. Then, a PD-L1 antibody-IL2 variant protein complex including anti-PD-L1 Fab-Fc and IL2 variant-Fc was obtained in the same manner as in Example 2-1, except that the first expression vector and the second expression vector were used.

### 3-2. Purification of PD-L1 Antibody-IL2 Variant Protein Complex

The PD-L1 antibody-IL2 variant 1 protein complex to the PD-L1 antibody-IL2 variant 15 protein complex obtained by culturing cell lines prepared in Example 3-1 were purified to obtain a high-purity substance.

First, the culture medium obtained in Example 3-1 was centrifuged to separate the medium from the cultured cells. Then, fine residues were removed from the PD-L1 antibody-IL2 variant protein complex contained in the separated medium using a 0.22 µm filter (Thermo Scientific Inc.). The medium after a filtering process was initially purified by Protein A-affinity chromatography (MabSelect PrismA, Cytiva). Specifically, after adding the filtered medium to a Protein A column stabilized by a phosphate buffer saline (pH 7.4), nonspecifically unbound proteins were washed using the same buffer, proteins specifically bound to the Protein A column were eluted using a buffer solution containing 0.05 M citric acid (pH 3.9), and the sample was neutralized to pH 7.2 by using 1 M Tris. Then, secondary purification was performed by cation exchange chromatography (Source30S, Cytiva) to remove impurities derived from residual substances in the sample separated by an affinity column. Specifically, after the sample eluted from the Protein A column and neutralized was titrated to pH 6.0 by adding 1 M citric acid, the sample was added to a Source30S column stabilized by 20 mM sodium phosphate (pH 6.0) to wash nonspecifically unbound proteins by using the same buffer, and PD-L1 antibody-IL2 variant protein complexes were obtained by elution using a buffer solution containing 0.3 M NaCl (pH 6.0) by an increasing gradient method. Then, after stabilizing the obtained PD-L1 antibody-IL2 variant protein complexes by using a phosphate buffer saline (pH 7.4), purity of the purified PD-L1 antibody-IL2 variant protein complexes was analyzed by size exclusion HPLC (TSK-3000SWxL, 7.8 mm x 30 cm, Tosoh Inc.).

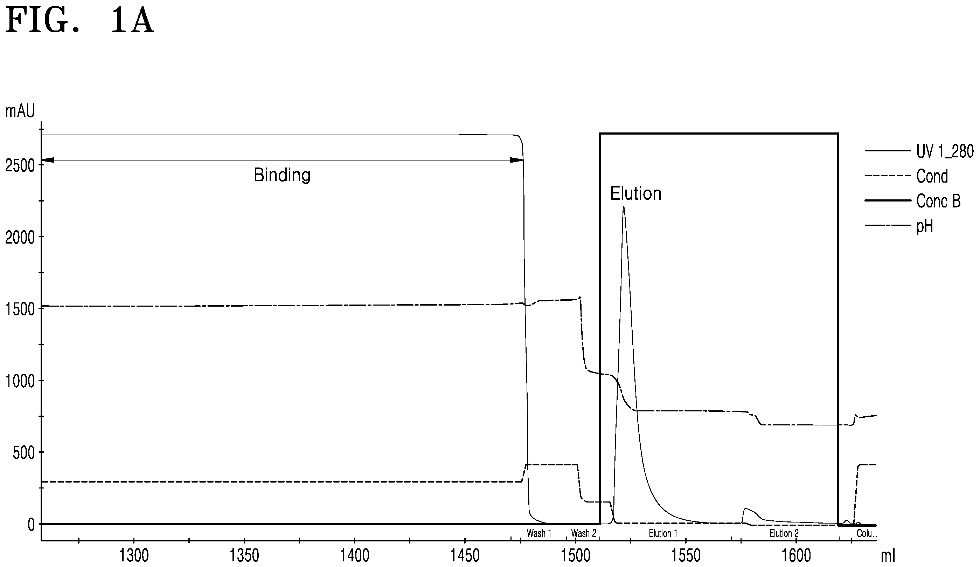

FIG. 1A shows results of purifying a PD-L1 antibody-IL2 variant 2 protein complex according to an embodiment by using a Protein A affinity column purification method.

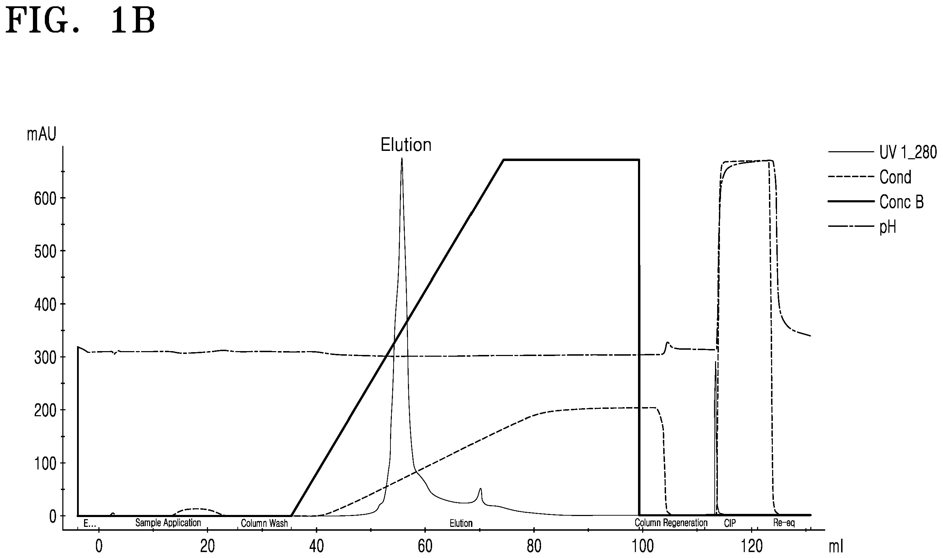

FIG. 1B shows results of purifying a PD-L1 antibody-IL2 variant 2 protein complex according to an embodiment by using a cation exchange resin column purification method.

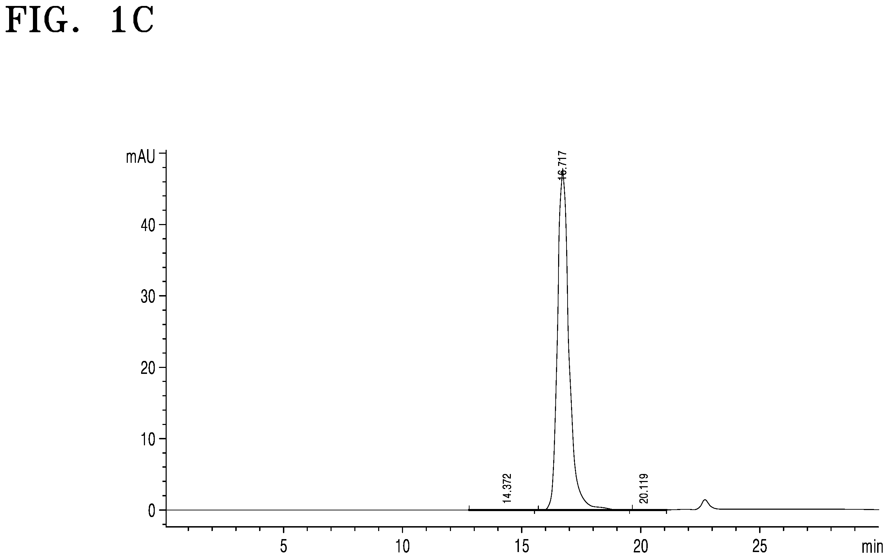

FIG. 1C shows results of analyzing purity of a PD-L1 antibody-IL2 variant 2 protein complex according to an embodiment by SE-HPLC analysis.

As a result, as shown in FIG. 1A, proteins specifically binding to the column were confirmed by the elution buffer used in Protein A affinity column purification. Specifically, the PD-L1 antibody-IL2 variant protein complex specifically binding to the column in 1500 to 1550 ml of the eluate could be confirmed.

In addition, as shown in FIG. 1B, it was confirmed that proteins nonspecifically binding to impurities remaining in the eluate used in the Protein A affinity column were removed.

In addition, as shown in FIG. 1C, it was confirmed that the purity of the PD-L1 antibody-IL2 variant 2 protein complex obtained by the Protein A affinity column purification and cation exchange resin purification was 99 % at a retention time of 16.717 minutes.

That is, it can be seen that the PD-L1 antibody-IL2 variant protein according to an embodiment is purified with high purity.

### 3-3. Identification of Binding Ability of PD-L1 Antibody-IL2 Variant Protein Complex to Receptor

Binding ability of the PD-L1 antibody-IL2 variant protein complex according to an embodiment to a receptor was identified by Surface Plasmon Resonance (SPR) analysis. Specifically, after fixing the human PD-L1 and the human IL2Rα, IL2Rβγ, and IL2Rαβγ to a CM5 sensor chip by covalent bonds, respectively, the PD-L1 antibody-IL2 variant 2 protein complex and the PD-L1 antibody-IL2 variant 4 protein complex prepared in Example 3-2 were applied at various concentrations (2-fold serial dilutions in a concentration range of 0.391 to 400 nM) to measure their binding kinetics to human PD-L1 and the IL2 receptor (IL2R). Then, binding affinity (KD) was calculated by using measured association constant (Ka) and dissociation constant (Kd). In this regard, SPR sensogram analysis was performed by BIAlogue kinetics evaluation software. Avelumab and Aldesleukin were used as positive controls, respectively, and the results are shown in Table 4 and Table 5.

**[Table 4]**

| Ligand | Experimental group | Binding affinity (KD, nM) |
|---|---|---|
| hPD-L1 | Avelumab | 0.12 |
| | PD-L1 antibody-IL2 variant 2 protein complex | 0.48 |
| | | |

**[Table 5]**

| Experimental group | Binding affinity (nM, SPR analysis) | | | |
|---|---|---|---|---|
| | Human IL2Rα | Human IL2Rβγ | Human IL2Rαβγ | βγ / αβγ |
| Aldesleukin | 15.8 | 0.25 | 0.05 | 5 |
| PD-L1antibody-IL2 variant 2 protein complex | No binding | 37.9 | 17.3 | 2.19 |
| PD-L1 antibody-IL2 variant 4 protein complex | No binding | 23.8 | 30.6 | 0.78 |

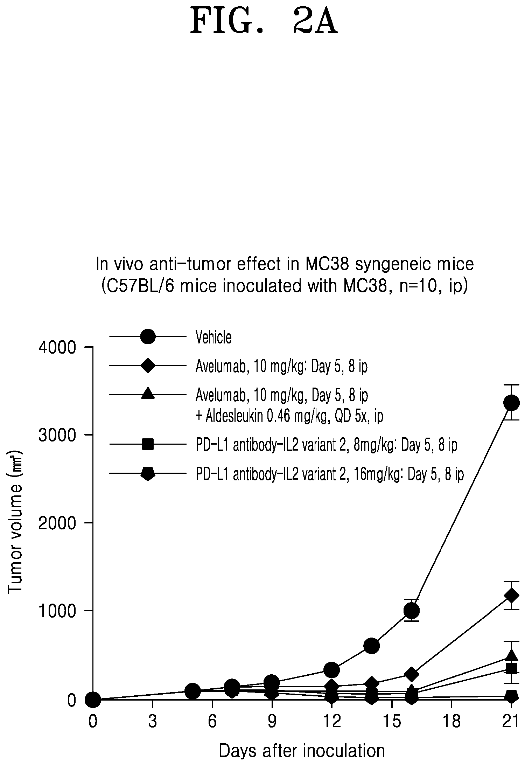

FIG. 2A shows results of identifying binding ability of a PD-L1 antibody-IL2 variant 2 protein complex according to an embodiment to human PD-L1 antigen.

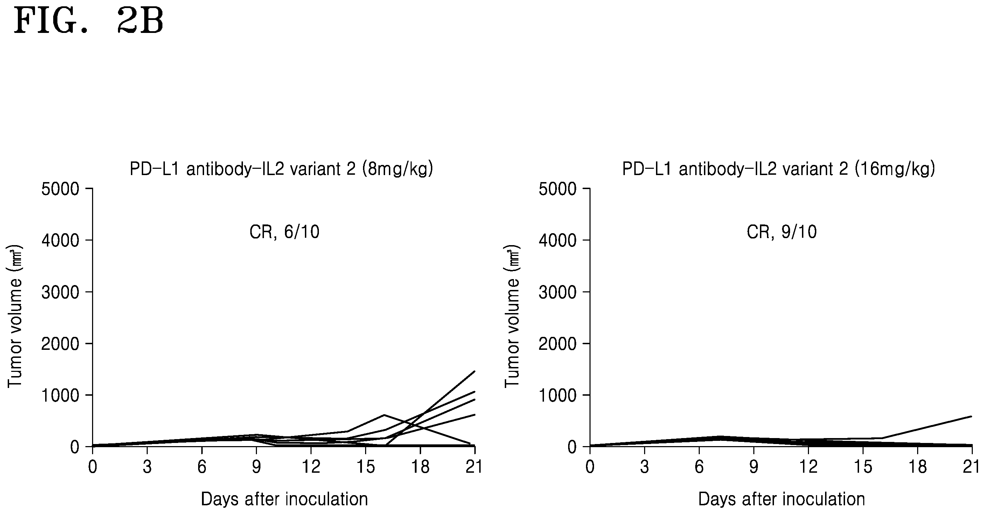

FIG. 2B shows results of identifying binding ability of Avelumab to human PD-L1 antigen.

As a result, as shown in FIG. 2A and Table 4, it was confirmed that the PD-L1 antibody-IL2 variant 2 protein complex prepared in Example 3-2 and the positive control bound to the human PD-L1 antigen. Specifically, the protein complex exhibited a binding ability of 0.48 nM, and the positive control exhibited a binding ability of 0.12 nM, which is about 4.1 times lower.

That is, while the positive control, as a monoclonal antibody, includes a bivalent anti-PD-L1 arm, the PD-L1 antibody-IL2 variant 2 protein complex includes a monovalent anti-PD-L1 arm, and thus there is a difference in binding ability therebetween.

In addition, as shown in Table 5, the PD-L1 antibody-IL2 variant 2 protein complex prepared in Example 2 showed a pattern of binding to IL2Rβγ and IL2Rαβγ with binding affinities of 37.9 nM and 17.3 nM, respectively, without binding to IL2Rα. In addition, the PD-L1 antibody-IL2 variant 2 protein complex showed a pattern of binding to IL2Rβγ and IL2Rαβγ with binding affinities of 23.8 nM and 30.6 nM, respectively, without binding to IL2Rα.

That is, it was confirmed that the PD-L1 antibody-IL2 variant 2 protein complex and the PD-L1 antibody-IL2 variant 4 protein complex according to an embodiment did not bind to the IL2 receptor α.

On the contrary, as shown in Table 5, it was confirmed that the positive control bound to IL2Rα, IL2Rβγ, and IL2Rαβγ with binding affinities of 35.7 nM, 1.55 nM, and 0.10 nM, respectively.

Meanwhile, it was confirmed that binding ability of the PD-L1 antibody-IL2 variant 2 protein complex and the PD-L1 antibody-IL2 variant 4 protein complex to IL2Rβγ decreased by about 24.5 times and 15.4 times, respectively, in comparison with the positive control. In addition, as a result of calculating βγ/αβγ ratios based on binding ability to IL2Rαβγ, the PD-L1 antibody-IL2 variant 2 protein complex and the PD-L1 antibody-IL2 variant 4 protein complex exhibited βγ/αβγ ratios of 2.19 and 0.78, respectively, and the positive control exhibited 15.5, and thus it was confirmed that the IL2R βγ/αβγ ratio of the PD-L1 antibody-IL2 variant protein complex significantly decreased in comparison with the positive control. That is, it can be seen that the PD-L1 antibody-IL2 variant 2 protein complex and the PD-L1 antibody-IL2 variant 4 protein complex not only did not bind to IL2Rα, but also have a weakened binding to IL2Rβγ.

Therefore, the PD-L1 antibody-IL2 variant protein complex according to an aspect is designed to induce high activation of CD8+ T cells relative to regulatory T cells, and it is characterized in that the binding ability to IL2Rα was removed and the binding ability to IL2Rβγ was regulated by substituting certain amino acids in order to selectively induce activation of CD8+T cells expressing IL2Rβγ.

### 3-4. Identification of Activity of Human Immune cell by PD-L1 Antibody-IL2 Variant Protein Complex

The effect of the PD-L1 antibody-IL2 variant 1 protein complex to the PD-L1 antibody-IL2 variant 15 protein complex according to an aspect on activation of human immune cells was identified. Specifically, peripheral blood mononuclear cells (StemCell Inc.) were reacted with specifically fluorescence-labeled effector CD8+ T cell (CD3+ and CD8+) and regulatory T cell (CD4+, CD25+, and FoxP3+) antibodies at 4 °C for 30 minutes in the dark, respectively. Then, after removing antibodies unbound to cells by centrifugation, peripheral blood mononuclear cells were treated with the PD-L1 antibody-IL2 variant 1 protein complex to the PD-L1 antibody-IL2 variant 15 protein complex prepared in Example 3-2 at respective concentrations (0.001 to 8000 nM), followed by reaction for 20 minutes at 37 °C in the dark and fixation using 1 mL of a fixation buffer (BD, U.S.) for 12 minutes. 1.5 mL of a Perm3 buffer (BD, U.S.) was treated to fluorescence-label phosphorylated STAT-5 in cytoplasm, followed by reaction for 40 minutes at 4 °C to allow fluorescent labels to penetrate into the cytoplasm. Then, phosphorylated STAT-5 proteins were fluorescence labeled, followed by reaction for 30 minutes, and ratios of cells in which STAT-5 was phosphorylated were identified in effector CD8+ T cells and regulatory T cells, respectively, by flow cytometry. Aldesleukin (Proleukin, Novartis, Switzerland) was used as a positive control, and the results are shown in Table 6.

**[Table 6]**

| | STAT5 phosphorylation (EC₅₀, nM) | |
|---|---|---|
| | Treq cell | CD8+T cell |
| Aldesleukin | 0.007 | 0.44 |
| PD-L1 antibody-IL2 comparison group protein complex | 0.01 | 0.22 |
| PD-L1 antibody-IL2 variant 1 protein complex | 0.12 | 0.3 |
| PD-L1 antibody-IL2 variant 2 protein complex | 0.59 | 0.33 |
| PD-L1 antibody-IL2 variant 3 protein complex | N.S | 682.7 |
| PD-L1 antibody-IL2 variant 4 protein complex | 0.9 | 0.4 |
| PD-L1 antibody-IL2 variant 5 protein complex | 1.45 | 0.68 |
| PD-L1 antibody-IL2 variant 6 protein complex | 0.28 | 0.28 |
| PD-L1 antibody-IL2 variant 7 protein complex | N.S | 404.2 |
| PD-L1 antibody-IL2 variant 8 protein complex | 18.11 | 52.15 |
| PD-L1 antibody-IL2 variant 9 protein complex | 0.38 | 0.29 |
| PD-L1 antibody-IL2 variant 10 protein complex | 1.78 | 1.01 |
| PD-L1 antibody-IL2 variant 11 protein complex | 10.6 | 1.71 |
| PD-L1 antibody-IL2 variant 12 protein complex | 0.27 | 0.37 |
| PD-L1 antibody-IL2 variant 13 protein complex | 0.45 | 0.49 |
| PD-L1 antibody-IL2 variant 14 protein complex | 0.75 | 0.53 |
| PD-L1 antibody-IL2 variant 15 protein complex | 11.57 | 3.25 |

| | | |
|---|---|---|
| * N.S: Not saturated at high concentration | | |

As a result, as shown in Table 6, the EC₅₀ value ratios of CD8+ T cell/regulatory T cell were 62.9 and 22 in the positive control and the PD-L1 antibody-IL2 comparison group, respectively, and the EC₅₀ value ratios of CD8+ T cell/regulatory T cell were 0.16 to 2.88 in the PD-L1 antibody-IL2 variant 1 protein complex to the PD-L1 antibody-IL2 variant 15 protein complex prepared in Example 3-2, respectively. That is, it was confirmed that the positive control and the PD-L1 antibody-IL2 comparison group more strongly activate regulatory T cells, but the PD-L1 antibody-IL2 variant 1 protein complex to the PD-L1 antibody-IL2 variant 15 protein complex prepared in Example 3-2 more selectively activate CD8+ T cells that are effector T cells relative to regulatory T cells.

Therefore, the PD-L1 antibody-IL2 variant protein complex according to an aspect may be involved in immune responses associated with anticancer effects by inducing the activity of the effector T cells more strongly than that of the regulatory T cells.

### 3-5. Identification of Anticancer Activity of PD-L1 Antibody-IL2 Variant Protein Complex (1)

Anticancer activity of the PD-L1 antibody-IL2 variant protein complex according to an embodiment was identified. Specifically, MC38 cells (1 X 10⁶ cell) were subcutaneously administered to C57BL/6 mice (Female, 6-week old, Jabio) in the flank at a dose of 0.2 mL/mouse to prepare a colorectal cancer syngeneic mouse model. 5 days after inoculation, the PD-L1 antibody-IL2 variant 2 protein complex prepared in Example 3-2 was intraperitoneally administered at doses of 8 mg/kg and 16 mg/kg Q2D x 2 times, respectively. After administering the complex, anticancer activities were compared by measuring tumor volumes of the mice (3 times/week). Avelumab (Merck, Germany), as a positive control, was intraperitoneally administered at a dose of 10 mg/kg (Q2D x twice), and Avelumab and Aldesleukin were co-administered (Aldesleukin 0.46 mg/kg, QD x 5 times, i.p.+ Avelumab 10 mg/kg, Day 5, 7, i.p,).

FIG. 2A shows results of comparing anticancer activities of a PD-L1 antibody-IL2 variant 2 protein complex according to an embodiment and a positive control.

FIG. 2B shows results of identifying cancer cell growth inhibiting effect and complete response activity of a PD-L1 antibody-IL2 variant 2 protein complex according to an embodiment on colorectal cancer syngeneic mice on the basis of subjects.

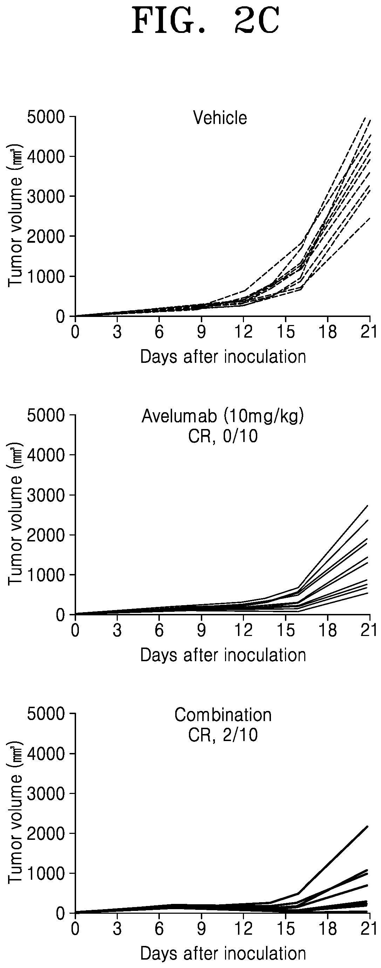

FIG. 2C shows results of identifying cancer cell growth inhibiting effect and complete response activity of a negative control and a positive control (Avelumab, and co-administration of Avelumab and Aldesleukin) on colorectal cancer syngeneic mice.

As a result, as shown in FIG. 2A, it was confirmed that tumor volumes increased over time after administration in the negative control (vehicle) and the positive control, but the PD-L1 antibody-IL2 variant 2 protein complex prepared in Example 3-2 exhibited a strong cancer cell growth inhibiting effect. Specifically, it was confirmed that most tumors disappeared at the dose of 16 mg/kg in the case of the PD-L1 antibody-IL2 variant 2 protein complex. In addition, unlike the PD-L1 antibody-IL2 comparison group protein complex, weight loss and death were not observed in the case of the PD-L1 antibody-IL2 variant 2 protein complex regardless of administered doses.

In addition, as shown in FIGS. 2B and 2C, tumor volumes increased over time after administration in the case of the negative control and the positive control, and it was confirmed that the number of mice showing the complete response (CR) was relatively small, as 0 to 2, out of 10 experimental animals in the same administration group. On the contrary, in the case of the PD-L1 antibody-IL2 variant 2 protein complex, the number of mice showing the complete response was 6 to 9 out of 10 experimental animals in the same administration group indicating an increase in a dose-dependent manner, and significantly greater anticancer activity was confirmed in comparison with the negative control and the positive control.

That is, superior anticancer activity was obtained by the PD-L1 antibody-IL2 variant protein complex according to an aspect in comparison with conventional antibody therapeutic agents, Avelumab and/or co-administration of Avelumab and Aldesleukin.

### 3-6. Identification of Anticancer Activity of PD-L1 Antibody-IL2 Variant Protein Complex (2)

Anticancer activity of the PD-L1 antibody-IL2 variant protein complex was identified. The anticancer activity was identified in the same manner as in Example 3-5, except that the PD-L1 antibody-IL2 variant 4 protein complex was used and Avelumab (Merck, Germany), as a positive control, was intraperitoneally administered (Q2D x twice) at a dose of 10 mg/kg.

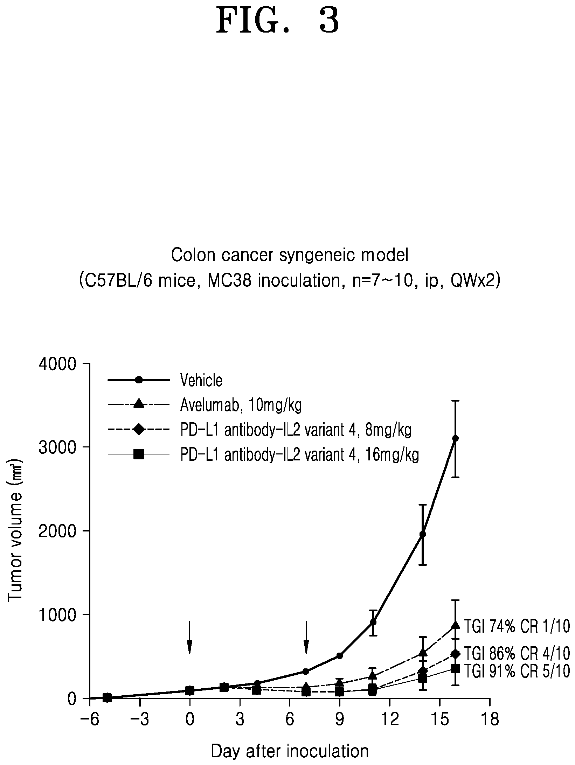

FIG. 3 shows results of identifying anticancer activity of a PD-L1 antibody-IL2 variant 4 protein complex according to an embodiment.

As a result, as shown in FIG. 3, it was confirmed that tumor volumes increased over time after administration in the negative control (vehicle), but the PD-L1 antibody-IL2 variant 4 protein complex prepared in Example 3-2 exhibited a strong cancer cell growth inhibiting effect in comparison with the negative control. Specifically, it was confirmed that changes in tumor volumes were significantly less in the case of the PD-L1 antibody-IL2 variant 4 protein complex at doses of 8 mg/kg and 16 mg/kg compared to administration of 10 mg/kg of the positive control. In addition, it was confirmed that the number of mice showing the complete response was 1 out of 10 experimental animals in the same administration group in the positive control, but the number of mice showing the complete response was 4 to 5 out of 10 experimental animals in the same administration group in the case of the PD-L1 antibody-IL2 variant 4 protein complex indicating that significant increase in anticancer activity compared to the positive control.

That is, the PD-L1 antibody-IL2 variant protein complex according to an aspect, which selectively increases the activity of effector T cells relative to regulatory T cells in comparison with conventional immunotherapy using antibodies, may be effectively used in prevention or treatment of various immunological diseases including cancer.

Therefore, the PD-L1 antibody-IL2 variant protein complex according to an aspect may provide therapeutic agent not only having superior anticancer activity, but also being safer than conventional immunotherapy for cancer by reducing side effects caused by IL2.

### Example 4. Preparation of MousePD-1 Antibody-IL2 Variant Protein Complex (a+b) and Identification of Activity

### 4-1. Cloning and Culture of MousePD-1 Antibody-IL2 Variant Protein Complex (a+b)

A protein complex (a+b) including anti-mousePD-1 Fab-Fc (hereinafter, referred to as "a") and IL2 variant-Fc (hereinafter, referred to as "b") was obtained in the same manner as in Example 3-1, except that the PD-1 antibody and the IL2 variants 1 and 4 prepared in Example 1 were used (See FIG. 4A).

### 4-2. Purification of MousePD-1 Antibody-IL2 Variant Protein Complex (a+b)

The mousePD-1 antibody-IL2 variant protein complex (a+b) obtained by culturing cell lines prepared in Example 4-1 was purified in the same manner as in Example 3-2 to obtain a high-purity substance.

### 4-3. Identification of Activity of Human Immune cell by MousePD-1 Antibody-IL2 Variant Protein Complex (a+b)

An experiment was performed in the same manner as in Example 3-4 to identify the effects of the mousePD-1 antibody-IL2 variant protein complex (a+b) obtained in Example 4-2 on activation of human immune cell.

**[Table 7]**

| | STAT5 phosphorylation (EC₅₀, nM) | |
|---|---|---|
| | Treq cell | CD8+ T cell |
| Aldesleukin | 0.029 | 0.42 |
| mousePD-1 antibody-IL2 variant 4 protein complex | 3.10 | 2.86 |

As a result, as shown in Table 7, it was confirmed that the CD8+ T cell/regulatory T cell ratios of the positive control and the mousePD-1 antibody-IL2 variant 4 protein complex (a+b) were 14.5 and 0.92, respectively. That is, it was confirmed that the positive control strongly activated regulatory T cells, but the mousePD-1 antibody-IL2 variant 4 protein complex (a+b) prepared in Example 4-2 selectively activated CD8+ T cells that are effector T cells rather than regulatory T cells.

Therefore, because the IL2 variant protein according to an aspect retains intrinsic properties thereof and promotes activity of effector T cells, even in the case of forming a complex with not only the PD-L1 antibody but also other Immune checkpoint inhibitors, it can be seen that the IL2 variant protein may be involved in immune responses.

### Example 5. Preparation of MousePD-1 Antibody-IL2 Variant Protein Complex (a+c) and Identification of Activity

### 5-1. Cloning and Culture of MousePD-1 Antibody-IL2 Variant Protein Complex (a+c)

Protein complexes including a mousePD-1 antibody and IL2 variant 1, 2, 3, 4, 7, 8, 14 and 15 were prepared. Specifically, a polynucleotide encoding a mousePD-1 antibody heavy chain variable region-constant region and a polynucleotide encoding a mousePD-1 antibody light chain constant region-light chain variable region were synthesized, respectively, by using the Invitrogen GeneArt Gene Synthesis service of ThermoFisher Scientific Inc. Then, a first expression vector expressing a mousePD-1 antibody was prepared by inserting the polynucleotides, respectively, into Avrll-Bstz171 enzyme site and EcoRV-PacI enzyme site of a pCHO1.0 expression vector. After synthesizing a polynucleotide encoding a complex including one of the IL2 variants 1, 2, 3, 4, 7, 8, 14, and 15 and a linker at the C-terminus of a polynucleotide encoding a mousePD-1 antibody heavy chain variable region-constant region by using the same service, the polynucleotide was inserted into AvrII-BstZ17I enzyme site of a pCHO1.0 expression vector and the mousePD-1 antibody light chain constant region-light chain variable region was inserted into EcoRV-PacI enzyme site to prepare a second expression vector. Then, a protein complex (a+c) including the anti-mousePD-1 Fab-Fc (a) and the anti-mousePD-1 Fab-Fc-C-terminus IL2 variant (hereinafter, referred to as "c") was obtained in the same manner as in Example 2-1 (See FIG. 4B).

### 5-2. Purification of MousePD-1 Antibody-IL2 Variant Protein Complex (a+c)

The mousePD-1 antibody-IL2 variant protein complex (a+c) obtained by culturing the cell lines prepared in Example 5-1 was purified to obtain a high-purity substance (purity of 98 % or more).

First, fine residues were removed and primary purification was performed in the same manner as in Example 3-2, except that the mousePD-1 antibody-IL2 variant protein complex (a+c) was used. Then, after adding the filtered medium to a Protein A column stabilized by a phosphate buffer saline (pH 7.4), nonspecifically unbound proteins were washed away using the same buffer and washed again using a buffer solution containing 0.05 M citric acid (pH 5.0). Then, proteins specifically bound to the Protein A column were eluted in a pH decreasing gradient method by using 0.02 M citric acid (pH 5.0 and pH 3.5), and the sample was neutralized to pH 7.2 using 1 M Tris.

Then, secondary purification was performed by a hydrophobic interaction column (Phenyl HP, Cytiva) to remove impurities derived from residual substances in the sample by an affinity column. Specifically, after the eluate from the Protein A column was neutralized to pH 7.0 by adding 1 M citric acid, the product was applied to a Phenyl HP column stabilized by 0.02 M sodium phosphate (pH 7.0) buffer and 0.8 M ammonium sulfate (pH 7.0). Nonspecifically unbound proteins were then washed away using the same buffer. Then, elution was performed in a salt concentration decreasing gradient method by using a buffer solution containing 0.02 M sodium phosphate (pH 7.0) to obtain the mousePD-1 antibody-IL2 variant protein complex (a+c). The obtained mousePD-1 antibody-IL2 variant protein complex (a+c) was stabilized by a phosphate buffer saline (pH 7.4) and purity was analyzed by using size exclusion HPLC (TSK-3000SWxL, 7.8 mm x 30 cm, Tosoh Inc.).

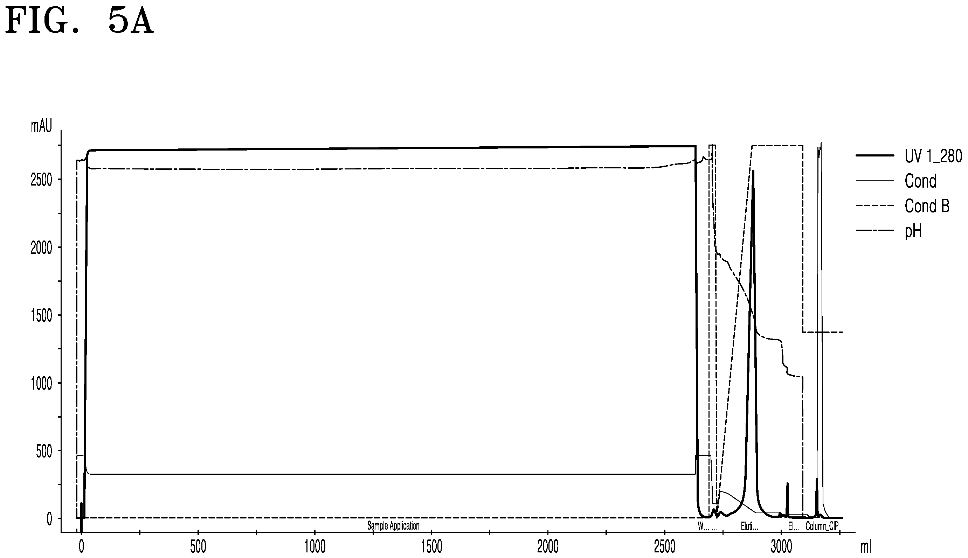

FIG. 5A shows results of purifying a mousePD-1 antibody-IL2 variant 14 protein complex (a+c) according to an embodiment by using a Protein A affinity column purification method.

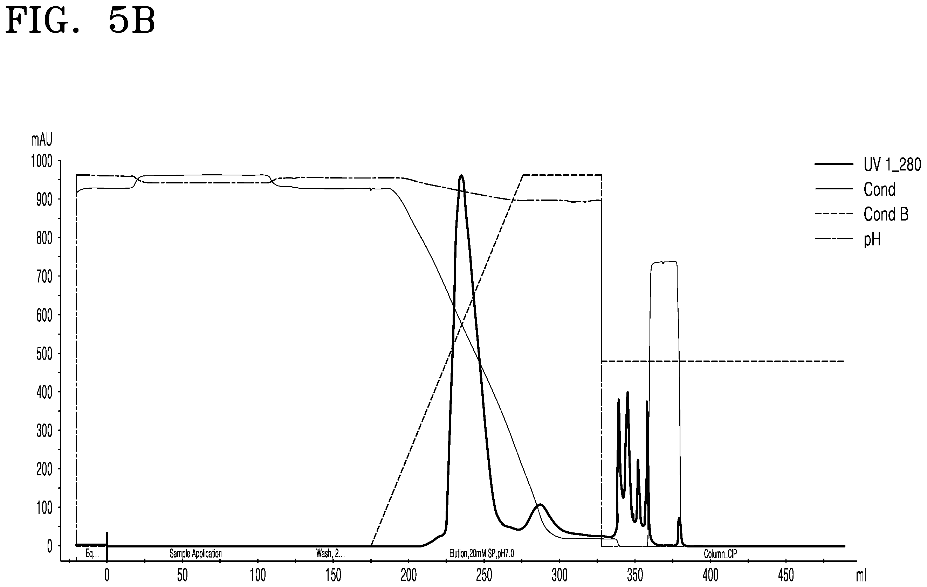

FIG. 5B shows results of purifying a mousePD-1 antibody-IL2 variant 14 protein complex (a+c) according to an embodiment by using a hydrophobic interaction column purification method.

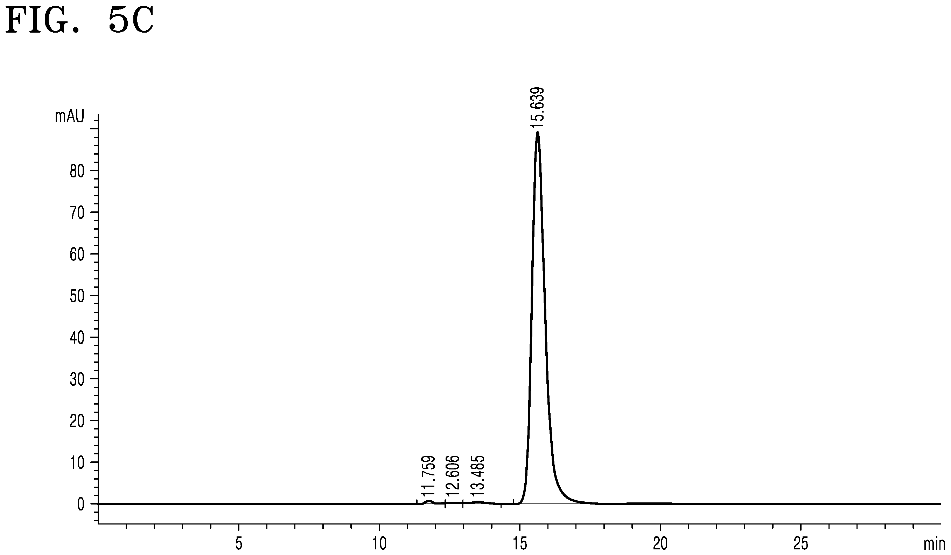

FIG. 5C shows results of analyzing purity of a mousePD-1 antibody-IL2 variant 14 protein complex (a+c) according to an embodiment.

As a result, as shown in FIG. 5A, proteins specifically bound to the column were able to be identified by the elution buffer used in the Protein A affinity column purification. Specifically, the mousePD-1 antibody-IL2 variant 14 protein complex (a+c) specifically binding to the column in 2800 to 2900 ml of the eluate was confirmed.

In addition, as shown in FIG. 5B, it was confirmed that proteins nonspecifically binding to impurities remaining in the eluate used in the Protein A affinity column were removed.

In addition, as shown in FIG. 5C, it was confirmed that the purity of the mousePD-1 antibody-IL2 variant 14 protein complex (a+c) obtained through all purification processes was 98.6 % at a retention time of 15.639 minutes.

That is, it can be seen that the mousePD-1 antibody-IL2 variant protein complex (a+c) according to an embodiment is purified with high purity.

### 5-3. Identification of Binding Ability of MousePD-1 Antibody-IL2 Variant Protein Complex (a+c) to Receptor

Binding ability of the mousePD-1 antibody-IL2 variant protein complex (a+c) to a receptor was identified in the same manner as in Example 3-3, except that the mousePD-1 antibody-IL2 variant 1 protein complex (a+c) and the mousePD-1 antibody-IL2 variant 14 protein complex (a+c) prepared in Example 5-2 were developed in 2-fold serial dilutions in a concentration range of 1.56 to 8000 nM.

**[Table 8]**

| Experimental group | Binding affinity (nM, SPR analysis) | | |
|---|---|---|---|
| | Human IL2Rα | Human IL2Rβγ | Human IL2Rαβγ |
| muPD-1 antibody-IL2 variant 1 protein complex | 5,610 | 4.51 | 1.01 |
| muPD-1 antibody-IL2 variant 14 protein complex | 6,290 | 35.3 | 25.7 |
| muPD-1 antibody-IL2 variant 14/ muPD-1 antibody-IL2 variant 1 (binding ability ralative ratio) | 1.1 | 7.8 | 25.4 |

As a result, as shown in Table 8, because the mousePD-1 antibody-IL2 variant 1 protein complex (a+c) and the mousePD-1 antibody-IL2 variant 14 protein complex (a+c) exhibited binding affinities of 5,610 nM and 6,290 nM to IL2Rβ respectively, similar levels of binding were confirmed. On the contrary, because the binding affinities to IL2Rβγ were 4.51 nM and 35.3 nM, respectively, it was confirmed that the binding ability of the mousePD-1 antibody-IL2 variant 14 protein complex (a+c) was 7.8-fold lower than that of the mousePD-1 antibody-IL2 variant 1 protein complex (a+c).

This indicates that the binding ability of the mousePD-1 antibody-IL2 variant 14 protein complex (a+c) to IL2Rγ decreased by about 7.8 times in comparison with the mousePD-1 antibody-IL2 variant 1 protein complex (a+c) in a state where the binding ability of the mousePD-1 antibody-IL2 variant 1 protein complex (a+c) to IL2Rβ was similar to that of the mousePD-1 antibody-IL2 variant 14 protein complex (a+c).

### 5-4. Identification of Activity of Human Immune cell by MousePD-1 Antibody-IL2 Variant Protein Complex (a+c)

The effect of the mousePD-1 antibody-IL2 variant protein complex (a+c) on activation of human immune cells was identified in the same manner as in Example 3-4, except that the mousePD-1 antibody-IL2 variant 1 protein complex, the mousePD-1 antibody-IL2 variant 2 protein complex, the mousePD-1 antibody-IL2 variant 3 protein complex, the mousePD-1 antibody-IL2 variant 4 protein complex, the mousePD-1 antibody-IL2 variant 7 protein complex, the mousePD-1 antibody-IL2 variant 8 protein complex, the mousePD-1 antibody-IL2 variant 14 protein complex, and the mousePD-1 antibody-IL2 variant 15 protein complex prepared in Example 5-2 and a mousePD-1 antibody-IL2 variant comparison group were used.

**[Table 9]**

| | STAT5 phosphorylation (EC₅₀, nM) | |
|---|---|---|
| | Treg cell | CD8+ T cell |
| Aldesleukin | 0.035±0.02 | 0.75±0.5 |
| muPD-1 antibody-IL2 variant 1 protein complex | 11.91±8.02 | 9.61 ±1.44 |
| muPD-1 antibody-IL2 variant 2 protein complex | 12.5±0.5 | 7.81±1.7 |
| muPD-1 antibody-IL2 variant 3 protein complex | 124.2±62.4 | 135.9±121.9 |
| muPD-1 antibody-IL2 variant 4 protein complex | 19.01±7.56 | 23.2±4.85 |
| muPD-1 antibody-IL2 variant 7 protein complex | 104.5 | 125.4 |
| muPD-1 antibody-IL2 variant 8 protein complex | 159.1 | 170.1 |
| muPD-1 antibody-IL2 variant 14 protein complex | 320.7±279.1 | 161.1±70.3 |
| muPD-1 antibody-IL2 variant 15 protein complex | 772.2±43.6 | 576.5±361.6 |

As a result, as shown in Table 9, it was confirmed that the EC₅₀ value ratio of CD8+ T cell/regulatory T cell was 21.4 in the case of the positive control (Aldesleukin), but 0.5 to 1.2 in the case of the mousePD-1 antibody-IL2 variant protein complex (a+c) prepared in Example 5-2. That is, it can be seen that the positive control more strongly activates regulatory T cells, but the mousePD-1 antibody-IL2 variant protein complex (a+c) according to an embodiment more selectively activates CD8+ T cells that are effector T cells relative to regulatory T cells. That is, it can be seen that intrinsic properties of the IL2 variant that selectively activate CD8+ T cells may be maintained even in a structure connected to the C-terminus of the FC region.

In addition, because the activity of Treg and CD8+ T cells by the mousePD-1 antibody-IL2 variant 1 protein complex (a+c) was 11.91±8.02 nM and 9.61±1.44 nM, respectively, while the activity of Treg and CD8+ T cells by the mousePD-1 antibody-IL2 variant 14 protein complex (a+c) was 320.7±279.1 nM and 161.1±70.3 nM, respectively, it was confirmed that the activity of the mousePD-1 antibody-IL2 variant 14 protein complex (a+c) to Treg and CD8+ T cells decreased in comparison with the mousePD-1 antibody-IL2 variant 1 protein complex (a+c). As confirmed in Example 5-3, this was caused by a decrease (about 7.8 times) in binding ability to IL2Rγ. In addition, the EC₅₀ value ratio of the effector T cell/regulatory T cell was 0.5, indicating that effector T cells (CD8+ T cells) were selectively activated relative to regulatory T cells.

Therefore, the mousePD-1 antibody-IL2 variant protein complex (a+c) according to an aspect may be involved in immune responses associated with anticancer effects by selectively activating effector T cells compared to regulatory T cells.

### 5-5. Identification of Anticancer Activity of MousePD-1 Antibody-IL2 Variant Protein Complex (a+c)

Anticancer activity of the mousePD-1 antibody-IL2 variant protein complex (a+c) according to an embodiment was identified. Specifically, MC38 cells (1 X 10⁶ cell) were subcutaneously administered to C57BL/6 mice (Female, 6-week old, KOATECH), acclimated for one week, in the right flank to prepare a colorectal cancer syngeneic mouse model. Then, when the size of tumors in mice reached about 70 to 120 mm², the mice were classified into groups of 3 to 6 mice each, and the mousePD-1 antibody-IL2 variant 14 protein complex (a+c) prepared in Example 5-2 was intraperitoneally administered once a week twice in total at a dose of 1, 3, 5, and 10 mg/kg. In this case, vehicle was used as a negative control, and 3 mg/kg of J43 (clone) that is anti-PD1 antibody and 3 mg/kg of J43 + 1.4 mg/kg of IL2 comparison group-Fc complex were used as positive controls. Then, tumor growth inhibiting effects by drug administration were observed after administration of a first drug until day 19.

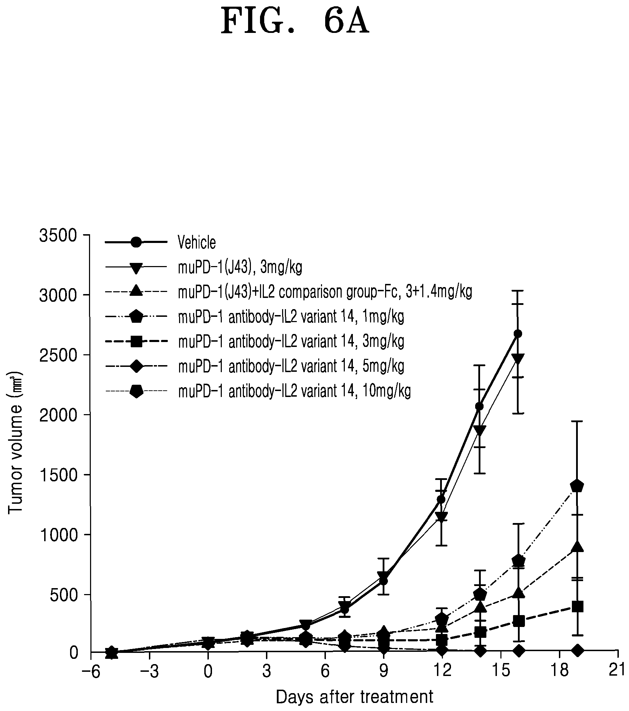

FIG. 6A shows results of comparing anticancer activities of a mousePD-1 antibody-IL2 variant 14 protein complex (a+c) according to an embodiment, the negative control, and the positive control in a colorectal cancer syngeneic mouse model.

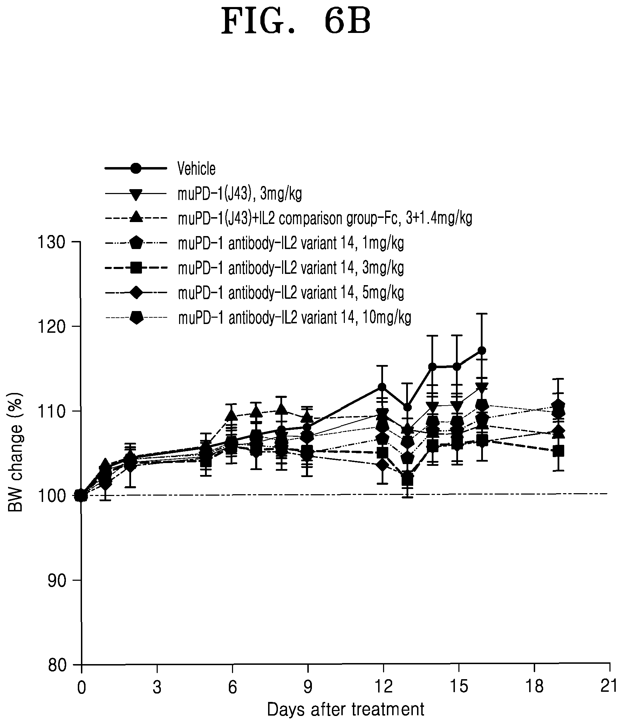

FIG. 6B shows results of comparing changes in body weights of a colorectal cancer syngeneic mouse model according to anticancer activity of a mousePD-1 antibody-IL2 variant 14 protein complex (a+c) according to an embodiment, a negative control, and a positive control.

As a result, as shown in FIGS. 6A and 6B, it was confirmed that tumor volumes increased over time after administration of the negative control and the positive control, but the mousePD-1 antibody-IL2 variant 14 protein complex (a+c) prepared in Example 5-2 exhibited a strong cancer cell growth inhibiting effect in dose-dependent manner in comparison with that immediately after administration. Particularly, in comparison with the positive control and the co-administration group, excellent cancer cell growth inhibiting effect was obtained in the case of administering the mousePD-1 antibody-IL2 variant 14 protein complex (a+c) at doses of 5 mg/kg and 10 mg/kg. That is, it was confirmed that the anticancer activity of the mousePD-1 antibody-IL2 variant 14 protein complex (a+c) was significantly greater than that of the PD-1 monoclonal antibody and co-administration of the IL2 comparison group-Fc protein complex.

Therefore, the mousePD-1 antibody-IL2 variant protein complex (a+c) according to an aspect, which specifically activates effector T cells in comparison with conventional immunotherapy for cancer, may be effectively used in prevention or treatment of various immunological diseases related to cancer.

### Example 6. Preparation of HumanPD-1 Antibody-IL2 Variant Protein Complex (A+C)

### 6-1. Cloning and Culture of HumanPD-1 Antibody-IL2 Variant Protein Complex (A+C)

Protein complexes including humanPD-1 antibody and each of the IL2 comparison group, the IL2 variant 1, and the IL2 variant 14 were prepared, respectively. Specifically, protein complexes (A+C) including the anti humanPD-1 Fab-Fc (hereinafter, referred to as "A") and the anti humanPD-1 Fab-Fc-C-terminus IL2 variant (hereinafter, referred to as "C") were obtained in the same manner as in Example 5-1, except that a polynucleotide encoding the humanPD-1 antibody heavy chain variable region-constant region and a polynucleotide encoding the humanPD-1 antibody light chain constant region-light chain variable region were used.

### 6-2. Purification of HumanPD-1 Antibody-IL2 Variant Protein Complex (A+C)

The humanPD-1 antibody-IL2 variant protein complex (A+C) obtained by culturing cell lines prepared in in Example 6-1 was purified in the same manner as in Example 5-2 and stabilized by a phosphate buffer saline (pH 7.4) to obtain high-purity humanPD-1 antibody-IL2 variant protein complex (A+C). Then, purity analysis was performed in the same manner as in Example 5-2.

### 6-3 Identification of Binding Ability of HumanPD-1 Antibody-IL2 Variant Protein Complex (A+C)

Binding ability of the humanPD-1 antibody-IL2 variant protein complex (A+C) to a receptor was identified in the same manner as in Example 3-3, except that the humanPD-1 antibody-IL2 comparison group protein complex (A+C), the humanPD-1 antibody-IL2 variant 1 protein complex (A+C), and the humanPD-1 antibody-IL2 variant 14 protein complex (A+C) prepared in Example 6-2 were developed in 2-fold serial dilution in a concentration range of 1.56 to 8000 nM. In this regard, Pembrolizumab was used as a positive control for human PD-1, and humanPD-1 antibody-IL2 variant 1 was used as a control, and the results are shown in Tables 10 and 11 below.

**[Table 10]**

| Ligand | Experimental group | Binding affinity (KD, nM) |
|---|---|---|
| hPD-1 | Pembrolizumab | 2.70 |
| | huPD-1 antibody-IL2 comparison group protein complex | 2.95 |
| | huPD-1 antibody-IL2 variant 1 protein complex | 2.25 |
| | huPD-1 antibody-IL2 variant 14 protein complex | 2.60 |
| | | |

**[Table 11]**

| Experimental group | Binding affinity (nM, SPR analysis) | | | |
|---|---|---|---|---|
| | Human IL2Rα | Human IL2Rβ | Human IL2Rβγ | Human IL2Rαβγ |
| huPD-1 antibody-IL2 comparison group protein complex | 105 | 6.506 | 3.08 | 0.023 |
| huPD-1 antibody-IL2 variant 1 protein complex | No binding | 5.462 | 2.92 | 0.782 |
| huPD-1 antibody-IL2 variant 14 protein complex | No binding | 5.611 | 68.3 | 25.5 |
| huPD-1 antibody-IL2 variant 14/ huPD-1 antibody-IL2 variant 1 (binding ability, ralative ratio) | - | 1.03 | 23.4 | 32.6 |

As a result, as shown in Table 10, it was confirmed that the huPD-1 antibody-IL2 comparison group protein complex (A+C), the humanPD-1 antibody-IL2 variant 1 protein complex (A+C), and the humanPD-1 antibody-IL2 variant 14 protein complex (A+C) prepared in Example 6-2 and the positive control exhibited binding abilities of 2.25 to 2.95 nM, which were similar binding abilities to human PD-1.

Meanwhile, as shown in Table 11, it was confirmed that the humanPD-1 antibody-IL2 variant 1 protein complex (A+C) and the humanPD-1 antibody-IL2 variant 14 protein complex (A+C) exhibited a pattern not reacting with IL2Rα. On the contrary, the humanPD-1 antibody-IL2 variant 1 protein complex (A+C) and the humanPD-1 antibody-IL2 variant 14 protein complex (A+C) exhibited binding abilities of 5.462 nM and 5.611 nM to IL2Rβ, respectively, which are similar levels of binding affinities, and it was confirmed that the humanPD-1 antibody-IL2 variant 1 protein complex (A+C) and the humanPD-1 antibody-IL2 variant 14 protein complex (A+C) bind to IL2Rβγ with binding affinities of 2.92 nM and 68.3 nM, respectively. Based on the above-described results, it was confirmed that the binding ability of the humanPD-1 antibody-IL2 variant 14 protein complex (A+C) to IL2Rβwas similar to that of the humanPD-1 antibody-IL2 variant 1 protein complex (A+C), but the binding ability thereof to IL2Rγ decreased. Specifically, it was confirmed that the binding ability of the humanPD-1 antibody-IL2 variant 14 protein complex (A+C) to IL2Rβγ and IL2Rαβγ decreased by about 23.4 times and 32.6 times in comparison with the humanPD-1 antibody-IL2 variant 1. That is, it can be seen that the humanPD-1 antibody-IL2 variant 1 protein complex (A+C) and the humanPD-1 antibody-IL2 variant 14 protein complex (A+C) not only did not bind to IL2Rα, but also have a weakened binding to IL2Rγ.

Therefore, the humanPD-1 antibody-IL2 variant protein complex (A+C) according to an aspect is used to induce high activation of CD8+ T cells relative to regulatory T cells, and it is characterized in that binding ability to IL2Rα is removed and binding ability to IL2Rγ is regulated by substituting certain amino acids in order to selectively induce activation of CD8+ T cells expressing IL2Rβγ.

The above description of the disclosure is for illustrative purposes, and those skilled in the art to which the disclosure belongs will be able to understand that the examples and embodiments can be easily modified without changing the technical idea or essential features of the disclosure. Therefore, it should be understood that the above examples are not limitative, but illustrative in all aspects.

## Claims

1. A protein comprising an IL2 variant, wherein the IL2 variant comprises glutamic acid (E) at position 35, alanine (A) at position 38, lysine (K) at position 42, and serine (S) at position 125.

2. The protein of claim 1, wherein the IL2 variant further comprises one or more amino acids selected from the group consisting of methionine (M), arginine (R), alanine (A), leucine (L), serine (S), phenylalanine (F), valine (V), isoleucine (I), glutamine (Q), tryptophan (W), asparagine (N), and threonine (T), at one or more positions selected from the group consisting of positions 18 and 19.

3. The protein of claim 2, wherein the IL2 variant comprises:
methionine (M) or arginine (R) at position 18; and
serine (S) at position 19.

4. The protein of claim 1, wherein the IL2 variant further comprises one or more amino acids selected from threonine (T) and isoleucine (I) at position 126.

5. The protein of claim 1, wherein the IL2 variant comprises amino acids selected from the group consisting of SEQ ID NOS: 1 to 15.

6. The protein of claim 1, wherein the protein comprises an Fc region linked by a linker or carrier.

7. A protein complex comprising: a first polypeptide including a first CH3 antibody constant region; and a second polypeptide including a second CH3 antibody constant region,
wherein the first polypeptide and the second polypeptide form a heterodimer,
an N-terminus of one or more of the first or second polypeptide comprises an antibody against an immune checkpoint inhibitor or an antigen-binding fragment thereof, or an antibody against a tumor associated antigen or an antigen-binding fragment thereof, and
one or more of the N-terminus or C-terminus of the first or second polypeptide comprises an IL2 variant.

8. The protein complex of claim 7, wherein the first CH3 antibody constant region comprises tryptophan (W) at position 366, and the second CH3 antibody constant region comprises serine (S) at position 366, alanine (A) at position 368, and valine (V) at position 407; and
the second CH3 antibody constant region comprises glycine (G) or phenylalanine (F) at position 351,
(wherein the positions of the amino acids are as described in the Kabat EU index).

9. The protein complex of claim 7, wherein the first CH3 antibody constant region comprises serine (S) at position 366, alanine (A) at position 368, and valine (V) at position 407, and the second CH3 antibody constant region comprises tryptophan (W) at position 366; and
the first CH3 antibody constant region comprises glycine (G) or phenylalanine (F) at position 351,
(wherein the positions of the amino acids are as described in the Kabat EU index).

10. The protein complex of claim 7, wherein the first CH3 antibody constant region comprises tryptophan (W) at position 366, and the second CH3 antibody constant region comprises glycine (G) at position 351, serine (S) at position 366, alanine (A) at position 368, and valine (V) at position 407; and
the first CH3 antibody constant region comprises phenylalanine (F) or tryptophan (W) at position 351,
(wherein the positions of the amino acids are as described in the Kabat EU index).

11. The protein complex of claim 7, wherein the first CH3 antibody constant region comprises glycine (G) at position 351, serine (S) at position 366, alanine (A) at position 368, and valine (V) at position 407, and the second CH3 antibody constant region comprises tryptophan (W) at position 366; and
the second CH3 antibody constant region comprises phenylalanine (F) or tryptophan (W) at position 351,
(wherein the positions of the amino acids are as described in the Kabat EU index).

12. The protein complex of claim 7, wherein the first CH3 antibody constant region comprises glycine (G) at position 351, serine (S) at position 366, alanine (A) at position 368, and valine (V) at position 407, and the second CH3 antibody constant region comprises tryptophan (W) at position 366; and
the second CH3 antibody constant region comprises phenylalanine (F) or tryptophan (W) at position 351,
(wherein the positions of the amino acids are as described in the Kabat EU index).

13. The protein complex of claim 7, wherein the immune checkpoint inhibitor is one or more selected from the group consisting of PD-L1, PD-1, LAG-3, VISTA, BTLA, TIM-3, TIGIT, and CTLA-4.

14. The protein complex of claim 7, wherein the tumor associated antigen is one or more selected from the group consisting of PD-L1, EGFR, HER-2, B7H3, GPC3, CEA, TROP, and PSMA.

15. The protein complex of claim 7, wherein the second polypeptide; and the IL2 variant are linked by at least one linker or carrier.

16. The protein complex of claim 15, wherein the linker comprises an amino acid sequence of SEQ ID NO: 18.

17. A polynucleotide encoding the protein or protein complex of any one of claims 1 to 16.

18. A method of preparing a protein or protein complex, the method comprising transforming a cell with an expression vector encoding the protein or protein complex of any one of claims 1 to 17.

19. A protein complex prepared by the method of claim 18 and comprising an IL2 variant.

20. A pharmaceutical composition for preventing or treating cancer, comprising the protein or protein complex of any one of claims 1 to 17 as an active ingredient.

21. A method of preventing or treating cancer, the method comprising administering the protein or protein complex of any one of claims 1 to 17 to a subject in need thereof.

22. A use of the protein or protein complex of any one of claims 1 to 17, for preparing an anticancer drug.
